# EUROPEAN PATENT SPECIFICATION

(11) **EP 4 437 983 B1**
(45) Date of publication and mention of the grant of the patent: **28.10.2026**
(21) Application number: 23210644.3
(22) Date of filing: 17.11.2023
(51) Int. Cl.: A61B 17/70

(54) **COUPLING DEVICE FOR COUPLING A ROD TO A BONE ANCHOR AND SYSTEM OF SUCH A COUPLING DEVICE AND AT LEAST TWO BONE ANCHORS**
KOPPLUNGSVORRICHTUNG ZUR KOPPLUNG EINER STANGE AN EINEN KNOCHENANKER UND SYSTEM EINER DERARTIGEN KOPPLUNGSVORRICHTUNG UND MINDESTENS ZWEI KNOCHENANKER
DISPOSITIF DE COUPLAGE POUR COUPLER UNE TIGE À UN ANCRAGE OSSEUX ET SYSTÈME D'UN TEL DISPOSITIF DE COUPLAGE ET D'AU MOINS DEUX ANCRAGES OSSEUX

(30) Priority: 30.03.2023 US 202363493238 P; 30.03.2023 EP 23165563
(43) Date of publication of application: 02.10.2024
(73) Proprietor: Biedermann Technologies GmbH & Co. KG, 78166 Donaueschingen (DE)
(72) Inventor: BIEDERMANN, Lutz, 78048 VS-Villingen (DE); BIEDERMANN, Timo, 78647 Trossingen (DE); DANNECKER, Berthold, 78112 St. Georgen (DE)
(74) Representative: Prüfer & Partner mbB Patentanwälte · Rechtsanwälte

(56) References cited:
- EP-A1- 3 673 844
- US-A1- 2007 090 238
- US-A1- 2019 046 242
- US-A1- 2019 105 085
- US-A1- 2023 008 092

## Description

The invention relates to a coupling device for coupling a rod to a bone anchor. The bone anchor can be of a first type or a second type which can be used in an interchangeable manner. The invention also relates to a system including such a coupling device and at least two bone anchors.

In spinal surgery modular bone anchors are known that can - at the choice of the user - be assembled from interchangeable parts to allow use of the bone anchor for different applications. For example, US 9,277,938 B2 describes a polyaxial bone anchoring system that includes a receiving part having an accommodation space, a first anchoring element having a first head, a sleeve-like insert piece configured to be arranged around a portion of the first head and to be arranged in the accommodation space, a second anchoring element having a second head, and a pressure member configured to exert pressure onto the first head or the second head, wherein the first bone anchoring element and the second bone anchoring element are configured to be interchangeably connectable to the receiving part. One of the bone anchoring elements is configured to pivot at a larger pivot angle compared to the other bone anchoring element. Hence, depending on the clinical application the first or the second bone anchoring element is combined with the receiving part.

US 2016/0113684 A1 describes a spinal implant system comprising a modular system including an array of members, such as, for example, receivers that can be selectively coupled to members, such as, for example, bone screw shafts. In some embodiments, the spinal implant system includes a crown configured to mate with a drive portion of a head of a screw shaft, the crown being configured to prevent the shaft from rotating along any axis and/or with any plane. In some embodiments, the crown is employed with a receiver having uni-axial screw functionality. In other embodiments, the crown is employed with a receiver having multi-axial screw functionality.

US 7,749,258 B2 describes a bone anchoring device in the form of a monoplanar bone anchoring device including a receiving part for receiving a rod, a pressure element and a bone anchoring element being movable relative to the receiving part in a limited angular range about the longitudinal axis of the receiving part, the angles lying in a single plane. The movement of the bone anchoring element relative to the receiving part is limited by a form fit connection comprising cooperating guiding surfaces at the head of the bone anchoring element and at the pressure element.

US 2007/090238 A1 describes a system for stabilizing a bony segment, comprising an anchor member, a receiver member, a retainer and a seat member, the retainer being positionable about a head of the anchor member within the receiver member. The retainer includes axially extending passages to receive pins provided at the head of the anchor member.

US 2019/046242 A1 describes a uniplanar bone anchor including a housing and a bone screw, wherein the lower region of the housing defines mating elements that mate with engaging elements on the bone screw to limit movement of the housing relative to the bone screw to a single plane.

EP 3 673 844 A1 describes a screw assembly comprising a screw coupled to a body member with a bushing provided in the body member and an insert coupled with the screw. The insert includes extensions that engage pockets in the body member to allow the body member to rotate relative to the screw only in one plane.

There is still a need to provide a simple, few parts containing modular bone anchoring system that allows to implement a polyaxial, an monoplanar and/or a monoaxial bone anchoring device.

Thus, it is the object to provide a coupling device that can be used in a modular bone anchoring device that enables a selectable functionality in terms of a polyaxial, monoplanar and/or a monoaxial design. It is also an object to provide a bone anchoring system that includes such a coupling device and at least two bone anchors that provide a different functionality to the bone anchoring device.

The object is solved by a coupling device according to claim 1 and by a bone anchoring system according claim 8. Further developments are given in the dependent claims. It shall be noted that the further developments of the coupling device can also be applied to the bone anchoring system and further developments of the bone anchoring system can also be applied to the coupling device.

According to an embodiment, a coupling device for coupling a rod to a first bone anchor of a first type or to a second bone anchor of a second type comprises a receiving part having a rod receiving portion for receiving the rod and a head receiving portion for pivotably receiving a head of the first bone anchor or a head of the second bone anchor such that a shank of the first or the second bone anchor can assume a plurality of angular positions relative to a pivot axis extending through the head receiving portion, and a pressure member that is configured to exert pressure on an inserted head of the first bone anchor or of the second bone anchor to clamp the head at a particular angular position. The coupling device comprises a restraining structure by means of which the coupling device is configured to cooperate with the first bone anchor such
that the first bone anchor is configured to pivot with respect to the pivot axis in a single plane or with the second bone anchor such that the second bone anchor is configured to pivot with respect to the pivot axis in multiple planes.

According to another embodiment, the coupling device is additionally or alternatively configured for coupling a rod to a third bone anchor of a third type. **In** this embodiment, the restraining structure of the coupling device is configured to cooperate with the third bone anchor such that the third bone anchor is prevented from pivoting relative to the coupling device and/or has a fixed orientation of its shank axis with respect to the pivot axis.

By selecting the first bone anchor and assembling it with the coupling device, the coupling device can be used as a monoplanar bone anchoring device. By selecting the second bone anchor and assembling it with the coupling device, the coupling device can be used as a polyaxial bone anchoring device. By selecting the third bone anchor, the coupling device can be used as a monoaxial bone anchoring device. Since the same coupling device can be used with different bone anchors, the surgeon has a more flexible and versatile choice of implant combinations while the number of parts per set is reduced.

**In** addition, as the receiving part and the pressure member are more expensive to manufacture compared to the bone anchor, the manufacturing costs and/or the costs for stock-holding can be decreased if the same coupling device can be used for more than one clinical application.

**In** a further embodiment, the restraining structure of the coupling device defines a first single plane in which the first bone anchor is configured to pivot with respect to the pivot axis, and also defines a second single plane in which the first bone anchor is configured to pivot with respect to the pivot axis, wherein the second single plane is different from the first single plane. The first bone anchor is configured to be selectively coupled to the coupling device in a first orientation so as to be pivotable in the first single plane or in a second orientation so as to be pivotable in the second single plane. Thus, a monoplanar coupling device having different pivot planes may be provided.

**In** a further embodiment, a first insert member can be added to the coupling device. The insert member is positionable in the pressure member and can assume at least two positions in which it exerts a different compression force onto a head of a bone anchor that is received in the coupling device. By means of this, the frictional force between the head and the pressure member can be adjusted such that it allows to provisionally lock the bone anchor relative to the coupling device in a particular angular position even if the rod is not yet inserted. This permits to carry out adjustment steps without the need of having the rod and/or a fixation member inserted in the rod channel.

In a still further embodiment, a second insert member may be provided that is configured to be inserted into the pressure member and that engages the head of a bone anchor in a form fit manner. By means of this, a monoaxial bone anchoring device can be implemented.

By selecting the first or the second insert, the choice of implant combinations can be further enlarged while still having fewer parts each of which are of simple design.

According to the embodiments, the bone anchoring device is of the bottom loading type, i.e. the bone anchor can be inserted from the bottom end of the receiving part. However, the bone anchoring device may also be of the top loading type, i.e. the bone anchor may be inserted from the top end of the receiving part.

A system of the coupling device and at least two bone anchors may include the coupling device and a first bone anchor of a first type and a second bone anchor of a second type, wherein the coupling device and the first bone anchor can be combined to a monoplanar bone anchoring device in which the bone anchor can pivot only in a single plane or wherein the coupling device can be combined with the second bone anchor to a polyaxial bone anchoring device in which the bone anchor can pivot in multiple planes.

In a further embodiment, a system of a coupling device and at least two bone anchors may include the coupling device and the first bone anchor of the first type and/or the second bone anchor of the second type, and a third bone anchor of a third type, wherein the coupling device can be combined with the first bone anchor to a monoplanar bone anchoring device in which the bone anchor can pivot only in a single plane or wherein the coupling device can be combined with the second bone anchor to a polyaxial bone anchoring device in which the bone anchor can pivot in multiple planes, or with the third bone anchor to form a monoaxial bone anchoring device in which the third bone anchor is prevented from pivoting relative to the coupling device.

According to a still further embodiment, a monoplanar bone anchoring device includes a coupling device for coupling a rod to a bone anchor, the coupling device comprising a receiving part having a rod receiving portion for receiving the rod and a head receiving portion for pivotably receiving a head of the bone anchor such that a shank of bone anchor can assume a plurality of angular positions relative to a pivot axis extending through the head receiving portion, and a pressure member that is configured to exert pressure on an inserted head to clamp the head at a particular angular position. The coupling device comprises a restraining structure by means of which the coupling device is configured to cooperate with the bone anchor such that the bone anchor can pivot with respect to the pivot axis in a single plane. The bone anchoring device further includes a bone anchor comprising a cooperating restraining structure that is configured to cooperate with the restraining structure of the coupling device, wherein the restraining structure of the coupling device comprises at least one recess and the restraining structure of the bone anchor comprises at least one protrusion configured to engage the recess such as to limit a pivoting movement of the bone anchor relative to the receiving part to a single plane.

According to a still further embodiment, a monoaxial bone anchoring device includes a coupling device for coupling a rod to a bone anchor, the coupling device comprising a receiving part having a rod receiving portion for receiving the rod and a head receiving portion for receiving a head of the bone anchor, and a pressure member that is configured to exert pressure on an inserted head. The coupling device comprises a restraining structure by means of which the coupling device is configured to cooperate with the bone anchor such that a shank of bone anchor can assume only one fixed angular position relative to a central axis extending through the head receiving portion, preferably such that a shank axis and the central axis are coincident. The bone anchoring device further includes a bone anchor comprising a mating restraining structure that is configured to cooperate with the restraining structure of the coupling device, wherein the restraining structure of the coupling device comprises at least two recesses and the restraining structure of the bone anchor comprises a corresponding number of protrusions configured to engage the recesses, the recesses and protrusions being shaped and arranged such as to to prevent a pivoting movement of the bone anchor relative to the receiving part.

According to a further embodiment an instrument is provided for inserting a bone anchor into bone, the bone anchor comprising a head and a shank, wherein the head has a substantially spherically-shaped outer surface portion and comprises a restraining structure at the outer surface that is configured to cooperate with a restraining structure of a coupling device, preferably wherein the restraining structure of the bone anchor is a projection comprising at least one wing that extends away from a shank axis of the shank, the wing preferably extending parallel to the shank axis along the spherically-shaped outer surface portion of the head, wherein the instrument comprises an engagement structure configured to engage the restraining structure of the head for coupling the instrument to the bone anchor so as to prevent rotation of the bone anchor relative to the instrument. The bone anchor being the first bone anchor and/or the third bone anchor described herein.

Since the instrument engages the bone anchor at the restraining structure, an additional engagement feature such as, for example, a polygonal engagement feature for engaging the head may not be required.

As used in the present specification and the appended claims, the term "rod" shall be understood as including any elongate member, regardless of the cross-sectional shape. Specifically, a spinal stabilization rod as used herein may have substantially circular, oval, or angular cross-section. Such a cross-section may vary along the length of the rod.

Further features and advantages of the invention will become apparent from the description of embodiments by mean of the accompanying drawings. In the drawings:
- Fig. 1:: shows a perspective exploded view of a system according to an embodiment including a coupling device with two bone anchors and two inserts member that can be used in an interchangeable manner.
- Fig. 2:: shows a perspective view of an embodiment of a bone anchoring device assembled from parts of the system of Fig. 1 in the assembled state.
- Fig. 3:: shows a perspective view from the top of an embodiment of the receiving part of the coupling device of Figs. 1 and 2.
- Fig. 4:: shows a perspective view from a bottom of the receiving part of Fig. 3.
- Fig. 5:: shows a top view of the receiving part of Figs. 3 and 4.
- Fig. 6:: shows a cross-sectional view of the receiving part of Figs. 3 to 5, and the cross-section taken along line A-A in Fig. 5.
- Fig. 7:: shows a perspective view from the top of an embodiment of a pressure member of the coupling device of Figs. 1 and 2.
- Fig. 8:: shows a perspective view from a bottom of a pressure member of Fig. 7.
- Fig. 9:: shows a top view of the pressure member of Figs. 7 and 8.
- Fig. 10:: shows a cross-sectional view of the pressure member of Figs. 7 to 9, the cross-section taken along line B-B in Fig. 9.
- Fig. 11:: shows another cross-sectional view of the pressure member of Figs. 7 to 10, the cross-section taken in a plane that is 90° rotated with respect to the plane of Figs. 9 and 10.
- Fig. 12:: shows a perspective view from the top of an embodiment of a first bone anchor of the system of Fig. 1.
- Fig. 13:: shows a top view of the first bone anchor of Fig. 12.
- Fig. 14:: shows a side view of the first bone anchor of Figs. 12 and 13.
- Fig. 15:: shows a perspective view from a top of a second bone anchor of the system of Fig. 1.
- Fig. 16:: shows a top view of the second bone anchor of Fig. 15.
- Fig. 17:: shows a cross-sectional view of the first bone anchor of Figs. 15 and 16, the cross-section taken along line D-D in Fig. 16.
- Fig. 18:: shows a perspective view from the bottom of a first insert member of the system of Fig. 1.
- Fig. 19:: shows a top view of the first insert member of Fig. 18.
- Fig. 20:: shows a cross-sectional view of the first insert member of Figs. 18 and 19, the cross-section taken along line F-F in Fig. 19.
- Fig. 21:: shows a perspective view from a top of a second insert member of the system of Fig. 1.
- Fig. 22:: shows a side view of the second insert member of Fig. 21.
- Fig 23:: shows a cross-sectional view of an embodiment of a bone anchoring device in the form of a polyaxial bone anchoring device using the coupling device and the second bone anchor of Fig. 1, wherein the cross-section is taken in a plane extending through the receiving part and extending perpendicular to a longitudinal axis of an inserted rod.
- Fig. 24:: shows a perspective view, partly in section, of a bone anchoring device assembled of the coupling member of the system of Fig. 1 and the first bone anchor in the form of an monoplanar bone anchoring device.
- Fig. 25:: shows a cross-sectional view of a bone anchoring device assembled from the coupling device of the system of Fig. 1 and the second bone anchor as well as the second insert member such that the resulting bone anchoring device is in the form of a monoaxial bone anchoring device, wherein the cross-section is taken in a plane including the central axis of the coupling device and extending perpendicular to a longitudinal axis of the rod channel.
- Fig. 26:: shows a cross-sectional view of a bone anchoring device in the form of a polyaxial bone anchoring device assembled from the coupling device of the system of Fig. 1 and the second bone anchor as well as the first insert member, wherein the cross-section is taken in a plane including the central axis of the coupling device and extending perpendicular to a longitudinal axis of the rod channel.
- Fig. 27:: shows a perspective view of a system according to a further embodiment, the system including a coupling device, the first bone anchor of the system of Fig. 1, and a third bone anchor, wherein the first and the third bone anchor can be used in an interchangeable manner.
- Fig. 28:: shows a perspective view from a bottom of the coupling device of Fig. 27.
- Fig. 29:: shows a perspective view from the top of the receiving part of the coupling device of Figs. 27 and 28.
- Fig. 30:: shows a perspective view from a bottom of the receiving part of Fig. 29.
- Fig. 31:: shows a top view of the receiving part of Figs. 29 and 30.
- Fig. 32:: shows a cross-sectional view of the receiving part of Figs. 29 to 31, and the cross-section taken along line G-G in Fig. 31.
- Fig. 33:: shows a perspective view from the top of a pressure member of the coupling device of Figs. 27 and 28.
- Fig. 34:: shows a perspective view from a bottom of the pressure member of Fig. 33.
- Fig. 35:: shows a top view of the pressure member of Figs. 33 and 34.
- Fig. 36:: shows a cross-sectional view of the pressure member of Figs. 33 to 35, the cross-section taken along line H-H in Fig. 35.
- Fig. 37:: shows a perspective view from the top of a third bone anchor of the system of Fig. 27 according to an embodiment.
- Fig. 38:: shows a perspective view from the top of the third bone anchor of Fig. 37.
- Fig. 39: shows a perspective view of a portion of an instrument according to an embodiment for inserting a bone anchor into bone, wherein the bone anchor comprises a restraining structure.
- Fig. 40: shows a perspective view of a portion of the instrument of Fig. 39 and the first bone anchor of Figs. 12 to 14.
- Fig. 41: shows a perspective view of a portion of the instrument and the first bone anchor of Fig. 40 in an engaged state.
- Fig. 42: shows a perspective view of a portion of an instrument according to a further embodiment for inserting a bone anchor into bone, wherein the bone anchor comprises a restraining structure.
- Fig. 43: shows a perspective view of a portion of the instrument of Fig. 42 and the third bone anchor of Figs. 37 and 38.

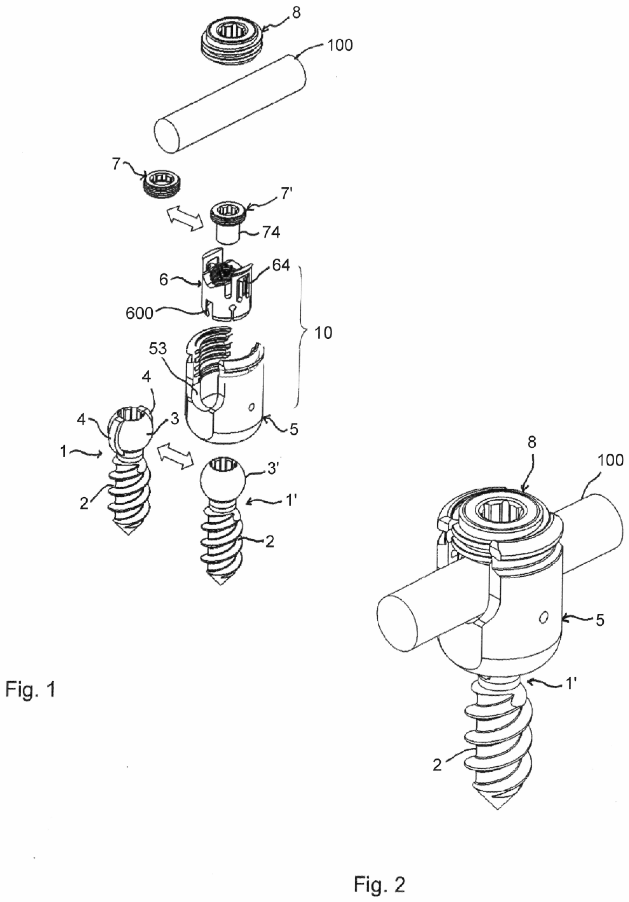

Referring to Fig. 1, a system of a coupling device and at least two bone anchors according to an embodiment includes at least a first bone anchor 1 of a first type and a second bone anchor 1' of a second type and a coupling device 10 that is connectable to the first bone anchor 1 or the second bone anchor 1'. When the coupling device 10 is assembled with the first bone anchor 1, the resulting bone anchoring device is an monoplanar bone anchoring device in which the first bone anchor 1 can pivot only in a single plane. When the coupling device is assembled with the second bone anchor, the resulting bone anchoring device is a polyaxial bone anchoring device in which the bone anchor can pivot in multiple planes. Thus, the first bone anchor 1 and the second bone anchor 1' may be interchangeably used together with one and the same coupling device 10

The coupling device 10 includes a receiving part 5 and a pressure member 6. The receiving part 5 is configured to accommodate a head 3 of the first bone anchor 1 or a head 3' of the second bone anchor 1' and a rod 100. The pressure member 6 is configured to be positionable in the receiving part 5 and to exert pressure into an inserted head. Optionally, the system may further include a first insert member 7 and/or a second insert member 7' that is positionable in the pressure member 6 and moveable thereto to act onto an inserted head. Optionally, the insert member 7 and the insert member 7' may be used interchangeably with the coupling device 10 assembled with the second bone anchor 1'. Furthermore, a fixation device in the form of, for example, a fixation screw 8 is provided for securing and fixing the rod 100 in the receiving part 5.

Fig. 2 shows the second bone anchor 1' assembled with the coupling device 10 and the rod 100 inserted and fixed by the fixation member 8.

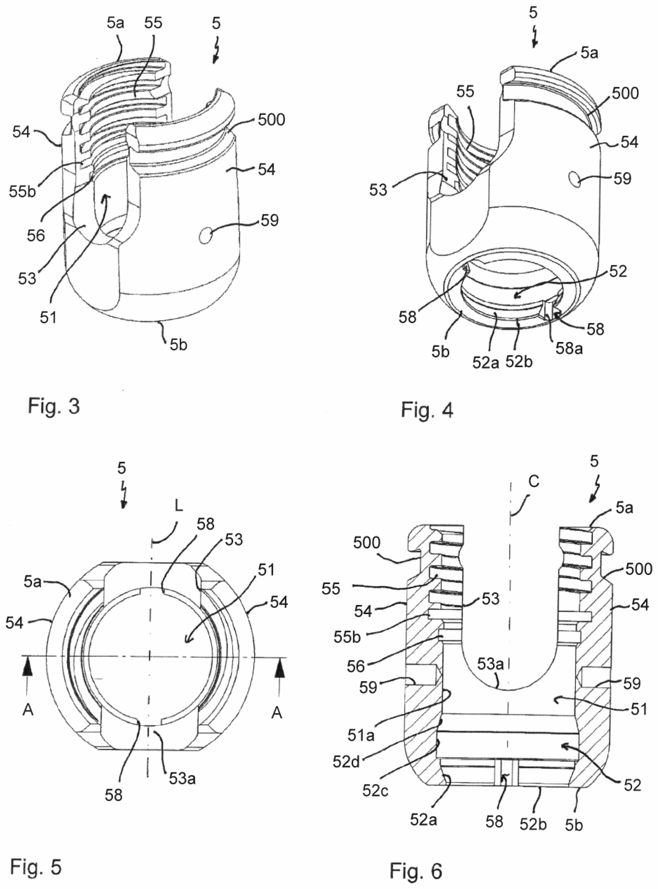

As depicted in greater detail in Figs. 3 to 6, the receiving part 5 may be substantially cylindrical and has a first end or top end 5a, a second end or bottom end 5b, and a passage 51 extending from the top end 5a towards the bottom end 5b and defining a longitudinal central axis C. Preferably, the receiving part is a monolithic piece. Adjacent to the bottom end 5b a head receiving portion 52 is formed for accommodating a head of one of the bone anchors and also a portion of the pressure member 6. The head receiving portion 52 comprises a narrowing portion 52a adjacent to the bottom end 5b that cooperates with a corresponding narrowing outer surface portion of the pressure member 6 to clamp the pressure member around an inserted head. At the bottom end 5b, the passage 51 provides an opening 52b that has a greatest width greater than a maximum width of a head of the bone anchors to be inserted. By means of this, the head can be inserted into the receiving part 5 from the bottom end 5b. The passage 51 widens from the narrowing portion 52a in the direction of the top end into a widened section 52c that provides space for a portion of the pressure member 6 to expand therein for entering the head. From the widened section 52c, a inversely narrowing section 52d extends towards the top end 5a and is followed by a portion 51a of the passage 51 with a substantially constant width, smaller than that of the widened section 52c.

The receiving part 5 further comprises a rod receiving portion that includes a substantially U-shaped recess 53 extending from the top end 5a to a distance therefrom. The substantially U-shaped recess 53 divides the receiving part 5 into two legs 54 and forms a channel for receiving the rod 100. An internal thread 55, for example a square thread, is provided at the receiving part 5 adjacent to the top end 5a for cooperating with the fixation member 8. At the lower end of the internal thread 55, there may be an undercut 55b. An upper edge of this undercut 55b may serve as an abutment for preventing the pressure member 6 to move upwards when the head is inserted. Moreover, a circumferential groove 56 may also be provided at the inner wall of each of the legs 54 at a position below the undercut 55b in a direction towards the bottom end 5b. The groove 56 may serve as a securing structure that holds the pressure member 6 in a position when the head is inserted and the pressure member engages the narrowing portion 52a of the head receiving portion so that the head cannot be removed.

The substantially U-shaped recess 53 defines a longitudinal axis L of the channel. As best shown in Figs. 4 to 6, two opposite recesses or slots 58 are formed at the bottom end 5b that extend from the bottom end 5b to approximately the widening section 52b of the receiving part 5. The recesses are offset from each other by 180° and are aligned with the channel axis L when seen in a top view as shown in Fig. 5. An inner contour of the recesses 58 may be substantially rectangular with rounded edges 58a. By means of the recesses 58, it is possible to insert the head 3 of the first bone anchor 1 from the bottom end 5b, as explained further below. Specifically, the recesses 58 define a restraining structure or a part of such a restraining structure, that restrains a pivoting motion of an inserted head of a bone anchor to a single plane including the central axis C.

In addition, at the center of each of the legs 54 in the circumferential direction, a transverse crimp bore 59 is formed. The crimp bores 59 may be located in the axial direction around approximately the height of a bottom 53a of the U-shaped recess 53. When the pressure member 6 is mounted to the receiving part 5 a crimping operation can be carried out that results in small protrusion at the inner wall of the legs 54 at the positions of the crimp bores 59 which protrude into the passage 51 and which are configured to engage a portion of the pressure member 6 to prevent rotation of the pressure member 6.

Lastly, close to the top end 5a, a circumferential groove 500 is formed on each of the legs that may serve as an engagement portion for an instrument.

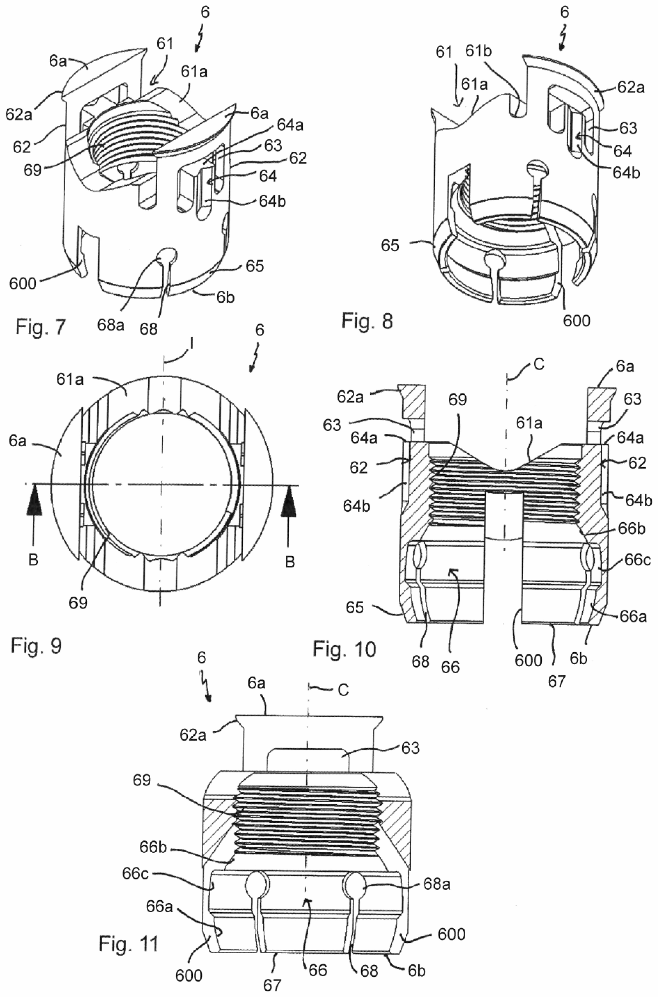

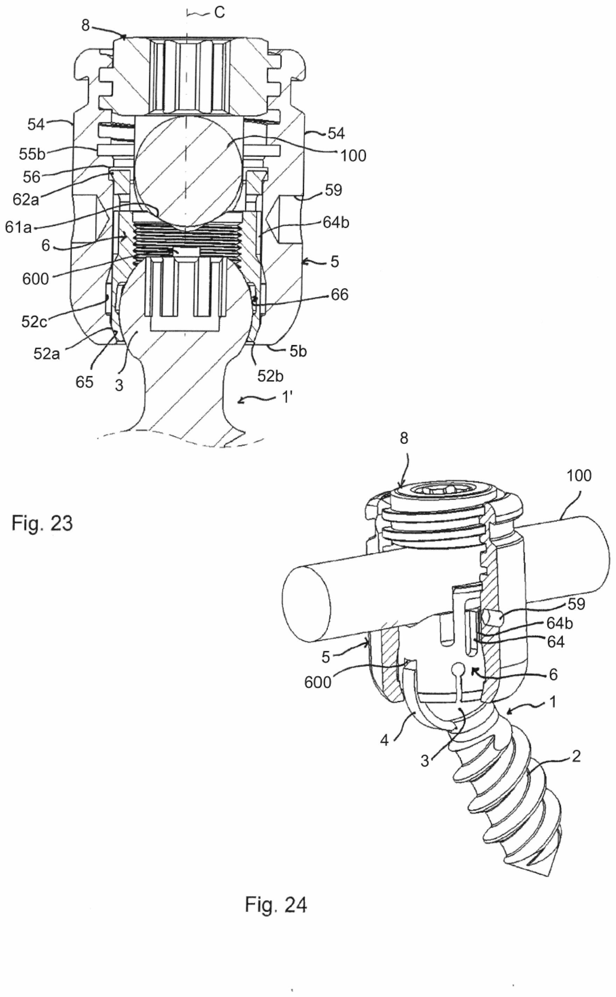

Referring in addition to Figs. 7 to 11, the pressure member 6 will be described in greater detail. Preferably, the pressure member 6 is a monolithic piece. It has a first end or top end 6a and a second end or bottom end 6b and may be substantially cylindrical with an outer diameter that allows it to move in the passage 51 of the receiving part 5. At the top end 6a, a rod receiving recess 61 is formed that provides a rod support surface 61a. The rod support surface 61a may have a substantially V-shaped cross-section with a longitudinal axis 1 extending substantially perpendicular to the cylinder axis of the pressure member 6 which coincides with the central axis C of the receiving part 5 when the pressure member 6 is positioned in the receiving part 5. A depth of the rod receiving recess 61 may be smaller than a diameter of the rod 100. Hence, when the rod 100 rests on the support surface 61a, it projects above the top end 6a of the pressure member 6, as shown for example in Figs. 23 and 24. The V-shape of the rod support surface 61a permits to use rods with different diameters.

Moreover, the rod receiving recess 61 is shaped such that two free legs 62 are formed that may be separated from the rod support surface 61a on each side by a groove 61b. By means of this, the legs 62 are slightly flexible in a direction transverse to the longitudinal axis of the rod support surface 61a. A free end of the legs 62 may have a radially protruding rim 62a at an upper surface which forms the first end 6a of the pressure member 6. The rim 62a is configured to engage the undercut 55b or the grooves 56 provided at the inner surface of the legs 54 of the receiving part 5 to secure an insertion position or a pre-locking position of the pressure member 6 in the receiving part 5. At the center of each of the legs 62 in the circumferential position, a cutout 63 is made in which a portion the legs 62 that forms a finger 64 extends such that a free end 64a of the 64 faces towards the top end 6a. In the outer surface of each of the finger 64 an axially extending groove 64b is formed that may extend slightly into the outer wall of the legs 62 below the cutout 63. The grooves 64b are configured to be engaged by the protrusions formed by crimping the crimp bores 59 provided in the receiving part 5.

Adjacent to the bottom end 6b of the pressure member 6 an outer surface portion 65 is provided that may be tapered, preferably conically-shaped, and that is configured to cooperate with the narrowing section 52a of the receiving part 5. Further, a hollow head receiving portion 66 is formed in the pressure member 6 that defines an opening 67 at the second end 6b for inserting a head of the bone anchor. The head receiving portion 66 may have a lower and an upper substantially spherical section 66a, 66b that are shaped so as to matingly receive a spherical head of the bone anchor therein. An intermediate section 66c has a greater inner width for facilitating the insertion of the head. In addition, a plurality of slits 68 are provided that extend completely through the pressure member 6 into the widened section 66c. The slits 68 are open to the second end 6b and are closed at their opposite end. At the closed end, the slits may comprise a widened section 68a that may, for example, be circular. As shown in Fig. 11, the closed end of the slits 68 may be located at an upper end of the widened section 66c of the head receiving portion 66. In the embodiment shown, four slits may be present, two on each side of the rod support surface 61a that may be positioned symmetrically with respect to the cutout 63 in the circumferential direction. However, the number and arrangement of the slits may vary, depending on the desired flexibility of the head receiving portion 66.

A coaxial threaded bore 69 that is coaxial with the central axis C extends through the rod support surface 61a into the head receiving portion 66 that permits access to an inserted head with an instrument. The bore 69 may be fully or partially threaded and is configured to receive the first insert 7 or the second insert 7' such that the insert can be advanced and retracted therein in the axial direction.

Lastly, two opposite recesses or slots 600 are formed in the lower portion of the pressure member 6. The recesses 600 are offset from each other by 180° and are in a top view as shown in Fig. 9 aligned with the longitudinal axis l of the rod support surface 61a . The recesses 600 are open to the second end 6b and closed towards the first end 6a. Preferably, they extend in the axial direction into the wall of the threaded bore 59. As best seen in Fig. 10, an inner contour of the recesses 600 may be rectangular. The size of the recesses 600 is such that a portion of the first bone anchor 1 can extend therein as further described below. When the pressure member 6 is mounted to the receiving part 5, the recesses 600 are aligned with the recesses 58 of the receiving part 5. Thus, the recesses 600 define a restraining structure that restrains a pivoting motion of the first bone anchor 1 to a single plane that includes the central axis C. The central axis C therefore defines the pivot axis.

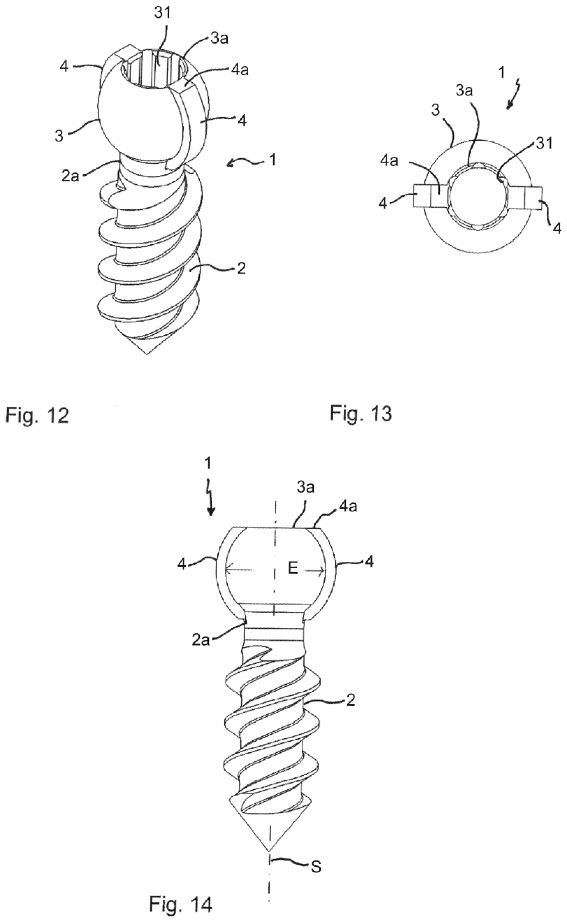

Referring further to Figs. 12 to 14, the first bone anchor 1 will be described in greater detail. The bone anchor 1 comprises a shank 2 with a shank axis S, wherein the shank is preferably fully or partially threaded, and a head 3 with a free end 3a. Between the head 3 and the shank 2 there may be a neck portion 2a. The head 3 has a spherical outer surface portion that includes the region with the greatest diameter E of the sphere. Preferably, the spherical outer surface portion is in the axial direction substantially symmetrical with respect to a plane including the plane of the greatest outer diameter E. In addition, the head 3 comprises two opposite wings 4 that extend along the spherical surface from the free end 3a of the head 3 up to the neck 2a. The wings 4 are offset by 180° and symmetrical with respect to a plane middle plane including the shank axis S and have each a shape of a spherical shell segment. An upper end 4a of the wings is flat and forms part of the free end 3a of the head 3. A width of the wings 4 is such that when the head 3 is received in the head receiving portion 66 of the pressure member 6, the wings 4 can extend into the recesses 600 and pivot therein. Thus, the wings 4 form a restraining structure or a part of a restraining structure that cooperates with the corresponding restraining structure in the form of the recesses 600 provided at the pressure member 6 to limit or restrain the pivoting motion of the head 3 in the head receiving portion 66 to a single plane. It shall be noted that the width of the wings 4 is such that when the head 3 passes through the lower opening 52b of the receiving part 5 during insertion, the wings 4 pass through the recesses 58 at the bottom end 5b of the receiving part 5.

Moreover, at the free end 3a, a tool engagement recess 31 is formed that may have a polygon or any other engagement structure such as, for example a torx-shaped or star-shaped or other axial groove-shaped structure suitable to screw the bone anchor 1 into bone. Additionally, a coaxial recess 32 with a bottom 32 that may have a slightly smaller diameter than the tool engagement recess 31 may be provided for receiving the second insert member 7' as explained below.

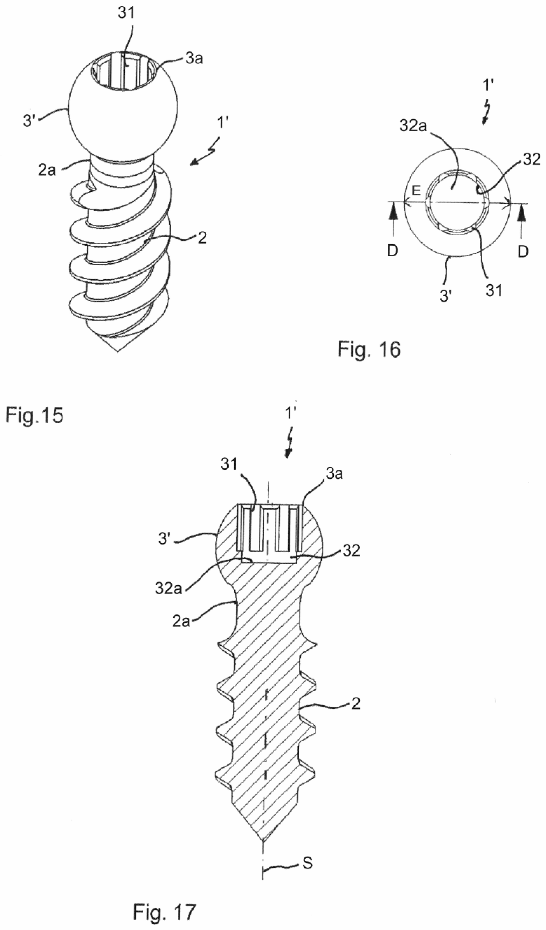

Referring to Figs. 15 to 17, the second bone anchor 1' will be described in greater detail. Parts and portions that are identical to the first bone anchor 1 are marked with the same reference numerals and the description thereof is not repeated. The second bone anchor 1' differs from the first bone anchor 1 in that the head 3' of the second bone anchor lacks the wings 4. Hence, the outer surface of the head 3' is uniformly spherically-shaped between the neck portion 2a and the free end surface 3a. As a result, when the second bone anchor 1' is inserted into the coupling device 10, the head 3' can freely pivot in the head receiving portion 66 of the pressure member 6 without being restrained. This means the second bone anchor 1' can pivot relative to the central axis C in multiple planes, even in an unlimited number of planes.

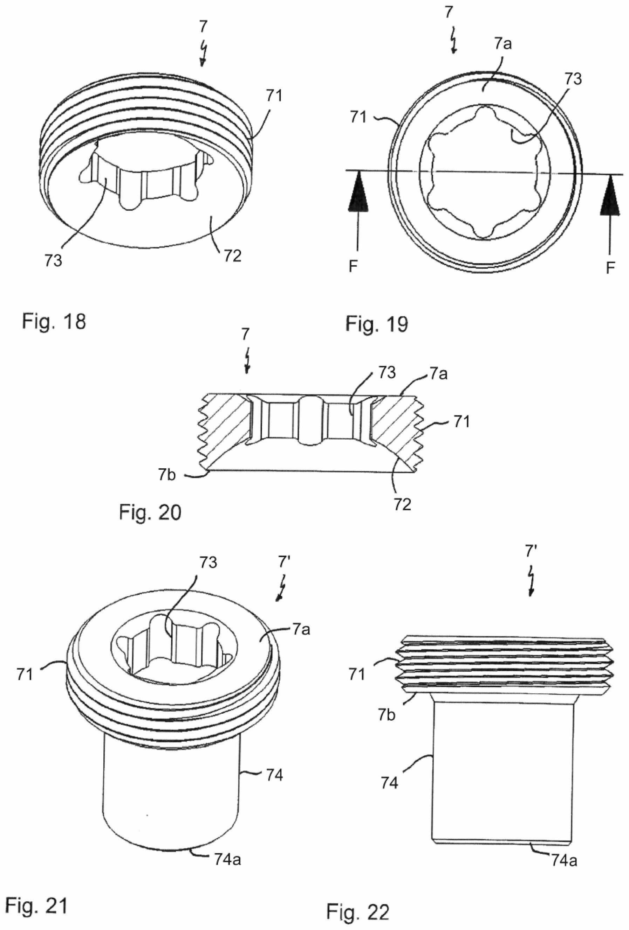

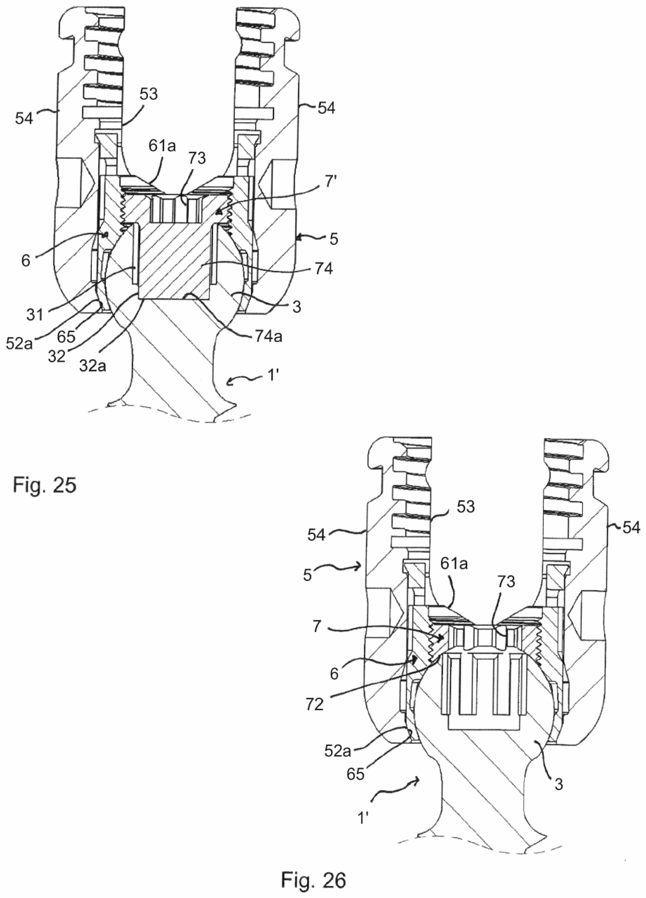

Referring to Figs. 18 to 20, the first insert member 7 is substantially a set-screw like member that has a first end or top end 7a and a second end or bottom end 7b. When the insert member 7 is assembled with the pressure member 6, the top end 7a of the insert member 7 faces towards the top end 6a of the pressure member 6. The outer surface 71 of the insert member 7 is threaded so that it can be screwed into the threaded bore 69 of the pressure member 6 and advanced therein, as can be seen in Fig. 26. Furthermore, on the bottom end 7b, a spherically-shaped recess 72 is formed that matches the shape of the spherical outer surface portion of the head 3' of the second bone anchor 1'. The size of the spherically-shaped recess 72 is such that the recess 72 is configured to cover at least a portion of the spherically-shaped outer surface portion of the head 3' when the head 3' is in the head receiving portion 66 of the pressure member 6. Moreover, a tool engagement recess 73 is provided at the top end 7a that may extend fully through the insert member 7 in the axial direction. The tool engagement recess 73 may have any shape suitable for engagement with a tool, such as, for example, a polygon shape or a torx-like or a star-like shape.

Turning now to Figs. 21 and 22, the second insert member 7' comprises an upper portion set-screw like portion with a threaded external surface 71 and with the tool engagement recess 73. Adjacent to the upper portion a lower portion 74 is formed that has the shape of a cylindrical post with a diameter smaller than that of the upper portion. In greater detail, the cylindrical post 74 has a diameter and a length such that when the head 3 of the first bone anchor 1 or the head 3' of the second bone anchor 1' is in the pressure member 6 and the second insert member 7' is positioned in the threaded bore 69, the cylindrical post 74 engages the recess 32 in the end surface of the head 3'. Due to this form-fit connection, the head of the bone anchor is prevented from pivoting at all. The post 74 may enter the recess 32 to such an extent that its bottom end 74b abuts against the bottom 32a of the recess 32 as shown in Fig. 25.

The parts and portions of the coupling device and the bone anchors, may be made of any material, preferably, however of titanium or stainless steel or of any bio-compatible metal or metal alloy or plastic material. For a bio-compatible alloy, a NiTi alloy, for example Nitinol, may be used. Other materials that can be used are magnesium or magnesium alloys. Bio-compatible plastic materials that can be used may be, for example, polyether ether ketone (PEEK) or poly-L-lactide acid (PLLA). The respective parts can be made of the same material or materials, or of different materials from another.

The coupling device may be pre-assembled. For assembly, the pressure member 6 is inserted into the receiving part from the top end 5a until the rim 62a enters the undercut 55b. Thereby, the recess 58 of the receiving part 5 and the recess 600 of the pressure member 6 are aligned and located between the respective legs 54 of the receiving part 5. A crimping step may be carried out with a crimping tool engaging the crimp bores 59 such that the protrusions that arise from the crimping enter the groove 64b at the finger 64. As a result, the pressure member 6 is prevented from rotating relative to the receiving part 5.

Prior to connecting the coupling device 10 to a bone anchor, the pressure member 6 is at an insertion position in which the head receiving portion 66 of the pressure member 6 can expand in the accommodation space of the head receiving portion 52 of the receiving part 5 when the head in inserted. In the insertion position, the pressure member 6 is prevented from moving towards the top end 5a of the receiving part 5, since the top end 6a of the pressure member 6 can abut against the upper edge of the undercut 55b. The pre-assembled coupling device 10 can be connected to a bone anchor either prior to inserting the bone anchor into bone or after the bone anchor has been inserted into bone. In both cases, the head of the bone anchor is inserted into the receiving part through the lower opening 52b and further moved into the head receiving portion 66 of the pressure member 6. Due to the slits 68 in the head receiving portion 66 the pressure member 6 snaps onto the head. Thereafter, the pressure member 6 is moved downward until the rim 62a enters the groove 56 on the inner wall of the receiving part. In this pre-locking position, an inserted head 3 can no longer be removed through the lower opening 52b.

The system of the coupling device and the at least two bone anchors comprising the first bone anchor 1 and the second bone anchor 1' allows to selectively combine the coupling device 10 with either the first bone anchor 1 or the second bone anchor 1'. Hence, using the same coupling device 10 either a monoplanar bone anchoring device or a polyaxial bone anchoring device can be generated.

As shown in Fig. 23, when the second bone anchor 1' is inserted into the coupling device, the second bone anchor 1' is configured to pivot around the central axis C in multiple planes. The restraining structure in the form of the recesses 58, 600 provided at the receiving part 5 and the pressure member 6, respectively, does not limit, i.e. has no influence on the pivoting motion of the second bone anchor 1'. After insertion of the rod 100, the fixation member 8 is screwed between the legs 55 until it presses via the rod 100 onto the pressure member 6 such that the pressure member is moved slightly downwards. The narrowing portion 52a of the receiving part and the narrowing portion 65 of the pressure member engage. By tightening the fixation member 8 the head 3' is locked in a particular angular position.

In the bone anchoring device shown in Fig. 24, the first bone anchor 1 is combined with the coupling device 10. For insertion, the head 3 is oriented with respect to the coupling device in such a manner that the wings 4 can engage the recesses 58 of the receiving part and 600 of the pressure member, respectively. By means of this, the pivoting motion of the first bone anchor 1 is restricted to a single plane. In the embodiment, due to the position of the restraining structure, the single plane is a plane defined by the central axis C and by the longitudinal axis L of the rod channel. After insertion of the rod 100 the fixation member 8 is screwed between the legs until it presses onto the rod 100 which in turn presses onto the pressure member to lock the head 3 in the coupling device.

Figs. 25 and 26 show combinations of the coupling device 10 with the first and the second bone anchor in a situation in which the rod has not yet been inserted. The insert members 7, 7' allow to carry out alignment and/or adjustment steps while the head can be locked without using the rod 100 and the fixation member 8.

In Fig. 25 the second bone anchor 1' is combined with the coupling device 10. In addition, the second insert member 7' is positioned within the threaded bore 69 of the pressure member 6 and screwed-in until it engages the recess 32 and abuts against the bottom 32a. A form-fit connection is achieved between the cylindrical post 74 and the head 3' of the second bone anchor 1' such that the second bone anchor 1' can no longer pivot. Simultaneously, the head 3' is pressed towards the lower portion of the receiving part which is narrowed by the pressure member 6. As a result, a monoaxial bone anchoring device is generated with the shank axis S being coaxial with the central axis C of the receiving part 5. The monoaxial functionality can be removed by removing the second insert member 7'. Alternatively it is possible to leave the second insert member 7' at its place, so that a permanent monoaxial bone anchoring device is generated.

The second insert member 7' can be used in a similar manner when the coupling device 10 is combined with the first bone anchor 1 to form a monoaxial bone anchoring device from a monoplanar bone anchoring device.

Fig. 26 shows a polyaxial bone anchoring device including the coupling device 10 and the second bone anchor 1' in which the first insert member 7 is used. The first insert member 7 is screwed into the threaded bore 69 until it contacts the head 3' that enters into the recess 72. By adjusting the axial position of the first insert member 7, the friction force exerted onto the head 3' can be adjusted. Hence, a friction force can be generated that maintains the second bone anchor 1' in a desired angular position without the need of having the rod and a fixation member inserted. The friction force may be adjusted such that it can be overcome by manually pivoting the receiving part relative to the bone anchor. Alternatively, the friction force may be adjusted such that the head in the coupling device is provisionally locked.

In both cases shown in Figs. 25 and 26, after adjusting the relative position of the bone anchor to the coupling device, the rod can be inserted and the head can be finally locked by tightening the fixation member 8.

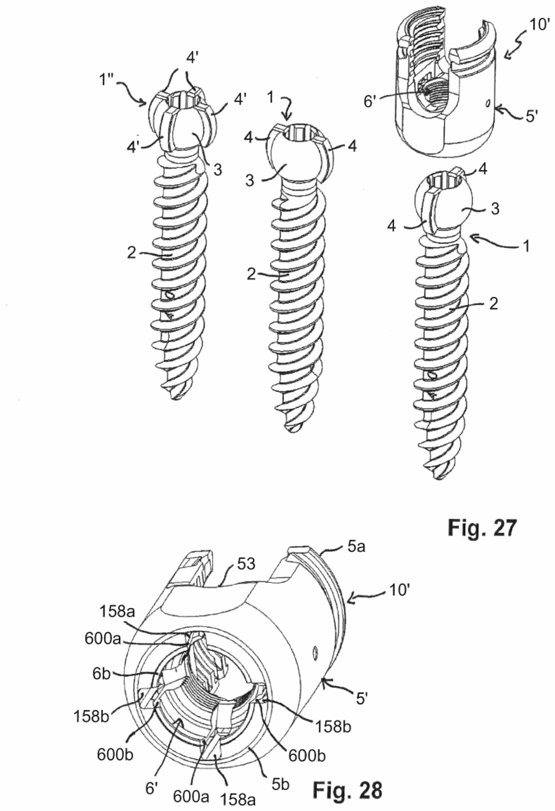

Next, a system of a coupling device and at least two bone anchors according to a further embodiment will be described with reference to Figs. 27 to 38. The system depicted in Fig. 27 includes at least the first bone anchor 1 of the first type described above and a third bone anchor 1" of a third type and a coupling device 10' according to a second embodiment. The coupling device 10' includes a receiving part 5' and a pressure member 6' and is selectively connectable to the first bone anchor 1 or the third bone anchor 1". Preferably the receiving part 5' is a monolithic piece and/or the pressure member 6' is a monolithic piece. Optionally, the system may further include the first insert member 7 and/or the second insert member 7' and/or a fixation device in the form of, for example, the fixation screw 8 for securing and fixing a rod 100 in the receiving part 5', as described above for the first embodiment (not shown in Fig. 27, see Fig. 1). When the coupling device 10' is assembled with the first bone anchor 1, the resulting bone anchoring device is an monoplanar bone anchoring device in which the first bone anchor 1 can pivot only in a single plane. Depending on the position or orientation in which the first bone anchor is assembled with the coupling device 10', the first bone anchor 1 is pivotable in a single first plane or in a single second plane different from the first plane, as explained in more detail below. When the coupling device is assembled with the third bone anchor 1", the resulting bone anchoring device is a monoaxial bone anchoring device in which the third bone anchor 1" is pivotably fixed with respect to the coupling device 10', i.e. is prevented from pivoting relative to the coupling device 10'. The first bone anchor 1 and the third bone anchor 1" may be interchangeably used together with one and the same coupling device 10'.

The coupling device 10' of the second embodiment depicted in Figs. 27 to 36 differs from the coupling device 10 of the first embodiment depicted in Figs. 1 to 26 with regard to the configuration of the restraining structure. In the Figures, parts and elements of the coupling device 10' of the second embodiment that are the same or similar to those of the coupling device 10 of the first embodiment are denoted with the same reference numerals, and a description thereof will be omitted in the following passages.

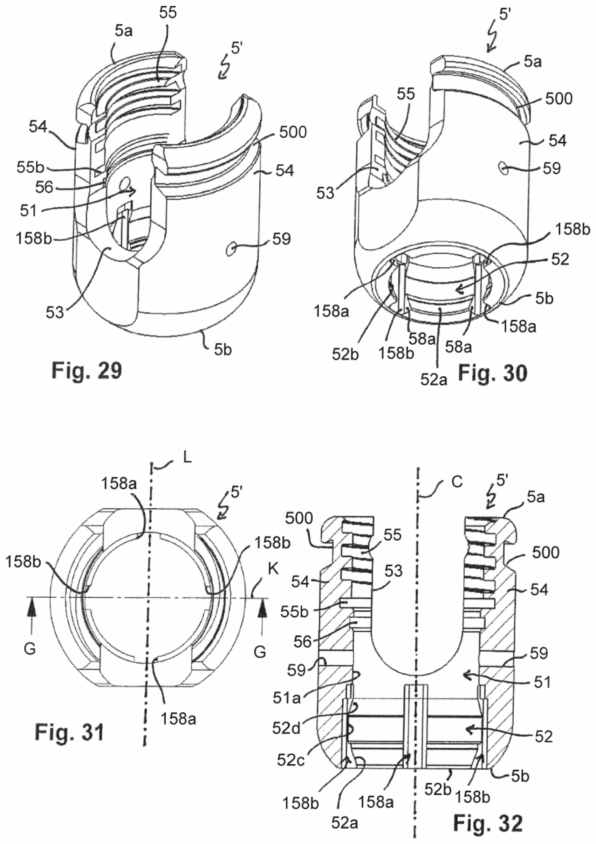

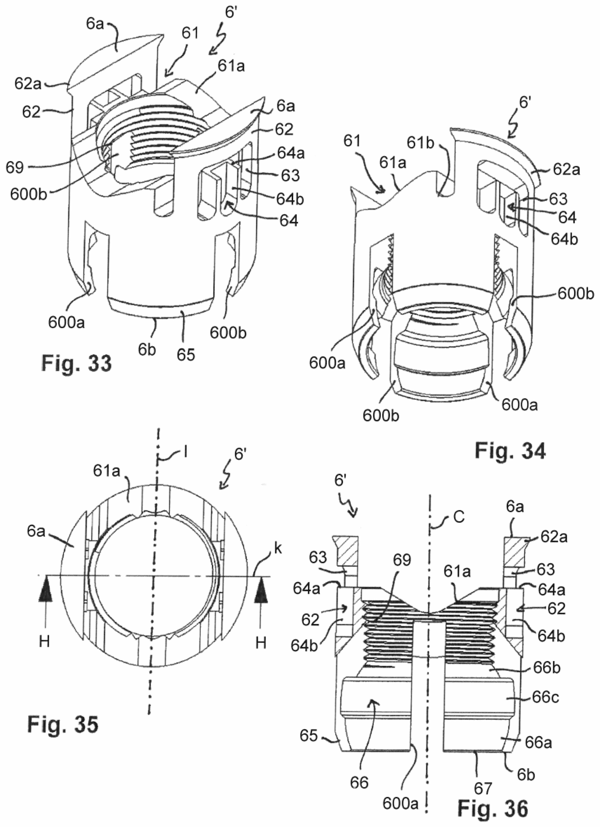

As can best be seen from Fig. 28, the coupling device 10' of the second embodiment differs from the coupling device 10 of the first embodiment specifically in that the restraining structure of the coupling device 10' includes a plurality of recesses or slots 600a, 600b formed in the lower portion of the pressure member 6' the number of which is such that at least two different distinct pivot planes can be defined. Also, a corresponding plurality of recesses or slots 158a, 158b is formed at the bottom end 5b of the receiving part 5'. The receiving part 5' will be described in more detail in the following passages with reference to Figs. 29 to 32 and the pressure member 6' with reference to Figs. 33 to 36.

The receiving part 5' comprises at its bottom end 5b four recesses or slots 158a, 158b, each recess extending from the bottom end 5b to approximately the portion 51a with a substantially constant width of the passage 51 of the receiving part 5'. The four recesses 158a, 158b are arranged in two opposing pairs of recesses. Specifically, two recesses 158a that form a first pair of recesses are offset from each other by 180° and are aligned with the channel axis L defined by the U-shaped recess 53 of the receiving part 5' when seen in a top view as shown in Fig. 31.

The other two recesses 158b that form a second pair of recesses are offset from each other by 180° and are aligned with an axis K that is perpendicular to the channel axis L when seen in the top view of Fig. 31. The recesses 158a of the first pair of recesses may be similar to or the same as the recesses 58 of the receiving part 5 of the first embodiment described above. Hence, in the present embodiment, the four recesses 158a, 158b are equally spaced at 90° intervals along the circumferential direction of the receiving part 5'. An inner contour of the recesses 158a, 158b may be substantially rectangular with rounded edges 58a (see Fig. 30).

The recesses 158a, 158b enable insertion of the head 3 of the first or the third bone anchor 1, 1" from the bottom end 5b of the receiving part 5', as explained above for the first embodiment. Specifically, the recesses 158a, 158b define a restraining structure or a part of a restraining structure that restrains a pivoting motion of an inserted head of the first bone anchor to a single plane including the central axis C, and/or prevents a pivoting motion of an inserted head of the third bone anchor 1" relative to the receiving part 5'.

The pressure member 6' comprises four recesses or slots 600a, 600b that are formed in the lower portion thereof. The four recesses 600a, 600b are arranged in two opposing pairs of recesses. Specifically, two recesses 600a that form a first pair of recesses are offset from each other by 180° and are aligned with the longitudinal axis l of the rod support surface 61a when seen in a top view as shown in Fig. 35. The other two recesses 600b that form a second pair of recesses are offset from each other by 180° and are aligned with an axis k that is perpendicular to the longitudinal axis l of the rod support surface 61a when seen in the top view of Fig. 35. Hence, in the present embodiment, the four recesses 600a, 600b are equally spaced at 90° intervals along the circumferential direction of the pressure member 6'. The recesses 600a of the first pair of recesses may be similar to or the same as the recesses 600 of the pressure member 6 of the first embodiment described above.

An inner contour of the recesses 600a, 600b may be rectangular (see Fig. 36). The recesses 600a, 600b are open to the second end 6b and closed towards the first end 6a of the pressure member 6'. They may extend in the axial direction into the wall of the threaded bore 69. The size of the recesses 600a, 600b is such that the wings 4 of the first bone anchor 1 can extend into the first pair of recesses 600a or into the second pair of recesses 600b and pivot therein, and the wings 4' of the third bone anchor 1" (see below) can extend into the first pair of recesses 600a and into the second pair of recesses 600b.

When the pressure member 6' is mounted to the receiving part 5', the recesses 600a, 600b of the pressure member 6' are aligned with the recesses 158a, 158b of the receiving part 5' as shown in Fig. 28. Thus, the recesses 600a, 600b define a restraining structure or part of a restraining structure. This restraining structure is on the one hand configured to restrain a pivoting motion of the first bone anchor 1 of the first type to a single plane that includes the central axis C. The central axis C may define the pivot axis. On the other hand, the restraining structure prevents a pivoting motion of an inserted head of the third bone anchor 1" relative to the central axis C.

As compared to the pressure member 6 of the first embodiment, the pressure member 6' of the second embodiment may be provided without slits 68 that render the head receiving portion 66 flexible. The four recesses 600a, 600b of the pressure member 6' may provide flexibility to the head receiving portion 66 so that the pressure member 6' can snap onto a head of a bone anchor during assembly of the bone anchor with the coupling device 10'.

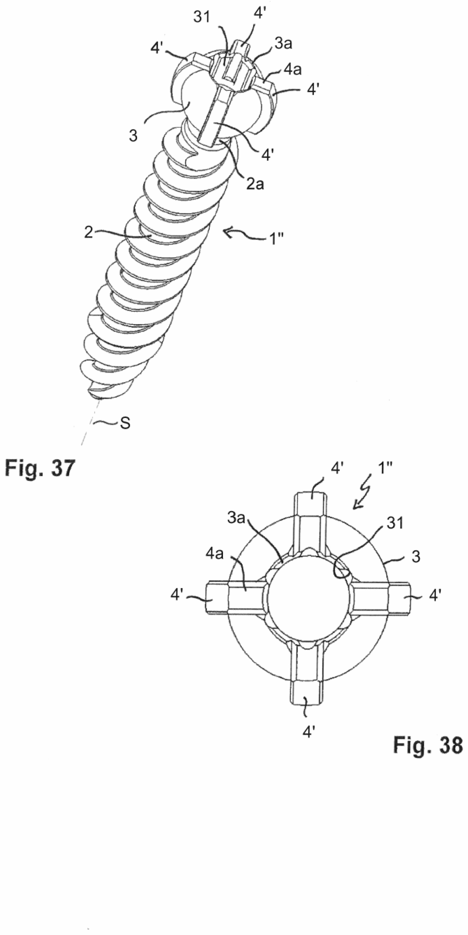

The third bone anchor 1" will now be described in more detail with further reference to Figs. 37 and 38. The third bone anchor 1" differs from the first bone anchor 1 described above in that it comprises four wings 4' forming a restraining structure. Specifically, the third bone anchor 1" comprises a first pair of wings on opposite sides of the head and a second pair of wings similar to the first pair of wings and offset from the first pair of wings by 90°. The four wings 4' of the third bone anchor are thus equally spaced at 90° intervals along the circumferential direction of the spherical outer surface portion of the head 3.

Similar to the wings 4 of the first bone anchor (see Figs. 12-14), the wings 4' of the third bone anchor 1" each have a shape of a spherical shell segment, i.e. have an elongate shape with a main extension direction of the wing 4' extending along the spherical outer surface portion of the head 3 in the direction of the shank axis S. A width of the wings 4' is such that when the head 3 is received in the head receiving portion 66 of the pressure member 6', the wings 4' can extend into the recesses 600a, 600b. Thus, the wings 4' form a restraining structure or a part of a restraining structure that cooperates with the corresponding restraining structure in the form of the recesses 600a, 600b provided at the pressure member 6' to prevent a pivoting motion of the head 3 in the head receiving portion 66. It shall be noted that the width of the wings 4' is such that when the head 3 passes through the lower opening 52b of the receiving part 5' during insertion, the wings 4' pass through the recesses 158a, 158b at the bottom end 5b of the receiving part 5'.

The steps of assembling the coupling device 10' of the second embodiment and inserting a head of a bone anchor are similar to those of the coupling device 10 of the first embodiment and will not be repeated here. The system of the coupling device 10' and the at least two bone anchors comprising the first bone anchor 1 and the third bone anchor 1" allows to selectively combine the coupling device 10' with either the first bone anchor 1 or the third bone anchor 1". Hence, using the same coupling device 10' either a monoplanar bone anchoring device, with the choice between two different pivot planes as explained below, or a monoaxial bone anchoring device can be obtained.

When the first bone anchor 1 is combined with the coupling device 10', the head 3 can be oriented with respect to the coupling device 10' in two positions during insertion. In a first position, the head 3 is oriented such that the wings 4 can engage the first pair of recesses 158a of the receiving part 5' and the first pair of recesses 600a of the pressure member 6'. In this position, the pivoting motion of the first bone anchor 1 is restricted to a single plane that is defined by the central axis C and by the longitudinal axis L of the rod channel. In a second position, the head 3 is oriented such that the wings 4 can engage the second pair of recesses 158b of the receiving part 5' and the second pair of recesses 600b of the pressure member 6'. In this position, the pivoting motion of the first bone anchor 1 is restricted to a single plane that is defined by the central axis C and by the axis K that is perpendicular to the longitudinal axis L of the rod channel.

When the third bone anchor 1" is combined with the coupling device 10', the head 3 is oriented such that each one of the four wings 4' can engage one of the recesses 158a, 158b of the receiving part 5' and the respectively aligned recess 600a, 600b of the pressure member 6'. In this position, any pivoting motion of the third bone anchor 1" with respect to the central axis C is prevented.

It should be noted that also the second bone anchor 1' described above (Figs. 15-17) can be combined with the coupling device 10' of the second embodiment. Because the second bone anchor 1' is free from any restraining structure, the second bone anchor 1' is configured to pivot around the central axis C in multiple planes when inserted into the coupling device 10'. The system described above with reference to Figs. 27 to 38 can therefore generally comprise the coupling device 10' and at least two bone anchors selected from a group consisting of the first bone anchor 1 of the first type, the second bone anchor 1' of the second type, and the third bone anchor 1" of the third type.

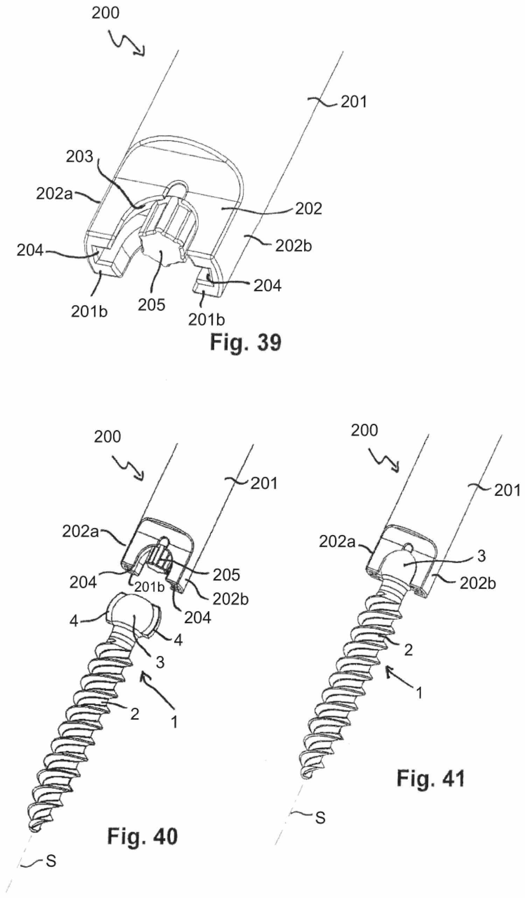

Next, a first embodiment of an instrument for inserting the first bone anchor 1 into bone will be described with additional reference to Figs. 39 to 41.

The instrument 200 comprises a substantially rod-shaped main portion 201 extending from a first end (not shown in the Figures) that may include a gripping portion (also not shown in the Figures) to a second end 201b. The main portion 201 may be provided with a flattened portion 202 adjacent to the second end 201b which may be generated by cutting away a part of the front section of the rod-shaped main portion 201. An U-shaped recess 203 is formed at the center of the flattened portion 202, which U-shaped recess 203 is open to the second end 201b of the instrument and shaped so as to receive at least a portion of the spherically-shaped outer surface portion of the head 3 of the bone anchor 1 therein. The recess 203 may have a shape that is complementary to the spherically-shaped portion of the head 3 at least in a portion thereof, so as to contact the inserted head 3 at least in a portion thereof (see Fig. 41). The U-shaped recess 203 divides the flattened portion 202 into two legs 202a, 202b.

Two grooves 204 are formed in the inner surfaces of the legs 202a, 202b that face the U-shaped recess 203 and are open to the second end 201b of the main portion 201. The size of the grooves 204 and their arrangement on the instrument 200 is such that they can engage the wings 4 of the first bone anchor 1 so as to transmit a force, in particular a torque, from the instrument 200 onto the bone anchor 1.

Optionally, a protrusion 205 is formed in the U-shaped recess 203 that extends between the two legs 202a, 202b towards the second end 201b of the instrument. An outer contour of the protrusion 205 is sized and shaped so as to engage the tool engagement recess 31 provided at the free end 3a of the head 3 of the first bone anchor 1 or the second bone anchor 1'. For example, the protrusion may have a polygon or any other shape such as, for example, a torx-shaped or star-shaped or other axial groove-shaped structure suitable to transmit a force, in particular a torque, from the instrument 200 onto the bone anchor 1. In a modification, the recess 203 may be spherically-shaped in such a manner that it covers only the upper portion of the head 3.

In order to insert, in particular screw, the bone anchor 1 into bone, the instrument 200 is combined with the bone anchor 1, as depicted in Figs. 40 and 41. As shown in Fig. 40, the instrument 200 is advanced towards the head 3 of the bone anchor 1 with its second end 201b facing the head 3 and the grooves 204 of the instrument 200 aligned with the wings 4 of the head 3. The grooves 204 then engage the wings 4 of the head 3 and may serve as a guiding structure to attach the instrument 200 to the head 3 of the bone anchor 1.

When the instrument 200 is attached to the head 3 of the bone anchor 1, as shown in Fig. 41, the protrusion 205 of the instrument engages the tool engagement recess 31 of the head 3 and the grooves 204 engage the wings 4 of the head 3. The head 3 is received within the U-shaped recess 203 and is laterally enclosed between the two legs 202a, 202b of the instrument 200. Thus, the bone anchor is prevented from rotating relative to the instrument and a force, in particular a torque, may be transmitted from the instrument 200 onto the bone anchor 1 to insert, in particular screw, the bone anchor 1 into bone. "Rotating" here refers to a rotation of the bone anchor around the shank axis S of the shank 2 relative to the main portion 201 of the instrument. The instrument 200 can also be used to insert the third bone anchor 1" or the second bone anchor 1" into bone.

It should be noted that the instrument 200 may be provided without the protrusion 205 and optionally the bone anchor 1 may be provided without the tool engagement recess 31, because the engagement of the grooves 204 with the wings 4 of the head 3 may be sufficient to transmit the force or torque from the instrument onto the head of the bone anchor. If the instrument 200 is provided without the protrusion 205, the head 3 may pivot relative to the main portion 201 of the instrument with the wings 4 of the head 3 received within the grooves 204. "Pivot" here refers to a pivoting motion of the shank axis S of the shank 2 relative to an axis of the main portion 201 of the instrument 200. This allows to couple the bone anchor 1 to the instrument 200 in a position where the shank axis S and the main portion 201 of the instrument are provided at an angle, i.e. not parallel. In some clinical situations such an angled engagement may be advantageous.

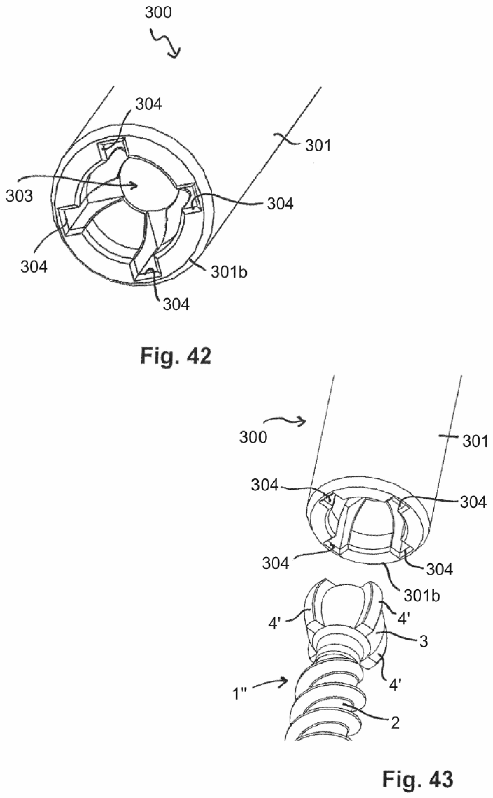

Figs. 42 and 43 show a second embodiment of an instrument 300 for inserting the first or third bone anchor 1, 1" into bone. The instrument 300 comprises a substantially rod-shaped main portion 301 extending from a first end (not shown in the Figures) that may include a gripping portion (also not shown in the Figures) to a second end 301b. A spherically-shaped recess 303 is formed at the second end 301b at the center of the main portion 301. The spherically-shaped recess 303 is open to the second end 301b and closed towards the first end of the instrument and shaped so as to receive at least a portion of the spherically-shaped outer surface portion of the head 3 of the third bone anchor 1" therein. The recess 303 may have a shape complementary to the spherically-shaped portion of the head 3 at least in a portion thereof, so as to contact the inserted head 3 at least in a portion thereof.

Four grooves 304 are formed in an inner surface of the main portion 301 in the region of the spherically-shaped recess 303 that are open to the second end 301b. The size of the grooves 304 and their arrangement on the instrument 300 is such that they can engage the four wings 4' of the third bone anchor 1" and/or the two wings 4 of the first bone anchor 1 so as to transmit a force, in particular a torque, from the instrument 300 onto the first or third bone anchor 1, 1", respectively. Specifically, in the present embodiment, the grooves 304 are equally spaced at 90° intervals along the circumferential direction of the spherically-shaped recess 303. Preferably, the grooves 304 are shaped so as to permit the wings 4 of the first bone anchor 1 to pivot therein, i.e. to allow for a pivoting motion of the shank axis S of the shank 2 relative to an axis of the main portion 301 of the instrument 300.

In order to insert, in particular screw, the third bone anchor 1" into bone, the instrument 300 is combined with the third bone anchor 1" by an engagement of the grooves 304 with the wings 4' of the head 3, as schematically depicted in Fig. 43.

When the instrument 300 is attached to the head 3 of the third bone anchor 1", each one of the four grooves 304 engages one of the wings 4' of the bone anchor 1" to establish a form-fit connection. By means of this, a force, in particular a torque, may be transmitted from the instrument 300 onto the third bone anchor 1" to insert, in particular screw, the bone anchor 1" into bone. The instrument 300 can also be used to insert the first bone anchor 1 into bone.

Modifications of the above described embodiments are conceivable. For example, the restraining structure in the receiving part and the pressure member can be at another position so that the single pivot plane can have an angle with respect to the channel axis. It may be also conceivable, that more than one restraining structures are provided at different locations such that several distinct pivot planes are provided one of which can be selected when inserting the first bone anchor. For example two, three or more distinct pivot planes can be implemented.

In the coupling device of the second embodiment, which is described above with reference to Figs. 27 to 36, the first pair of recesses 158a and 600a at the receiving part and pressure member define, together with the pivot axis or central axis C, a first plane in which the monoplanar pivoting motion of the first bone anchor 1 takes place, and the second pair of recesses 158b and 600b define, together with the pivot axis or central axis C, a second plane in which the monoplanar pivoting motion of the first bone anchor 1 takes place. By selecting the position of the first and/or second pair of recesses when manufacturing the coupling device, the single pivot plane of the first bone anchor can be accordingly defined.

The first and/or third bone anchor may have wings or another restraining structure which have a distance from the free end of the head. In this case the first insert member can also be used with the first bone anchor to achieve a friction force between the insert and the head for the monoplanar bone anchoring device.

In a further modification, the receiving part and the pressure member may be designed for top loading of the bone anchor. In such a case, the receiving part may have adjacent to the bottom end a seat portion for directly and pivotably receiving the head of the bone anchor. The pressure member may be shaped so as to exert pressure only from the top onto an inserted head.

For all embodiments it is possible that the shape of the head receiving portion of the coupling device is such that the pivot axis is angled with respect to the central axis C so as to provide a greater pivot angle to one side compared to an opposite side.

It shall be noted that particular shapes of the elements are not limited to the specific shape shown in the drawings but may vary.

The bone anchor is not limited to have a threaded shank. All types of bone anchors that are suitable for anchoring in bone or vertebrae may be used.

Instead of the fixation device described other known fixation devices may be implemented, such as, for example, a bayonet fixation device or a two part fixation device that allows the independent fixation of the rod and the head.

## Claims

1. A coupling device for coupling a rod to a first bone anchor (1) of a first type or to a second bone anchor (1') of a second type, the coupling device (10, 10') comprising:
a receiving part (5, 5') having a rod receiving portion (53) for receiving the rod (100) and a head receiving portion (52) for pivotably receiving a head (3) of the first bone anchor (1) or a head (3') of the second bone anchor (1') such that a shank (2) of the first or the second bone anchor can assume a plurality of angular positions relative to a pivot axis (C) extending through the head receiving portion (52), and
a pressure member (6, 6') that is configured to exert pressure on an inserted head (3, 3') of the first bone anchor (1) or of the second bone anchor (1') to clamp the head (3, 3') at a particular angular position,
wherein the coupling device (10, 10') comprises a restraining structure (58, 158a, 158b, 600, 600a, 600b) by means of which the coupling device (10, 10') is configured to cooperate with the first bone anchor (1) such that the first bone anchor (1) is configured to pivot with respect to the pivot axis (C) in a single plane, or with the second bone anchor (1') such that the second bone anchor (1') is configured to pivot with respect to the pivot axis (C) in multiple planes,
**characterized in that** the restraining structure comprises a first recess (600, 600a) provided in the pressure member (6, 6') that is configured to cooperate with a matching restraining structure (4) of the first bone anchor (1) to allow pivoting of the first bone anchor (1) in the single plane, and the restraining structure comprises a second recess (58, 158a, 158b) on the receiving part (5, 5'), that corresponds to the first recess on the pressure member and which is configured to cooperate with the first bone anchor (1).

2. The coupling device of claim 1, wherein the pressure member (6, 6') comprises a head receiving portion (66) enclosing an inserted head (3, 3') of the first bone anchor (1) or of the second bone anchor (1') from the side, the head receiving portion (66) defining a lower opening (67) for passing through the shank (2), wherein the restraining structure (600, 600a) is adjacent to the lower opening (67).

3. The coupling device of claim 1 or 2, wherein the pressure member (6, 6') defines a support surface (61a) for the rod (100) and wherein the restraining structure (600, 600a) is aligned with a longitudinal axis (I) of the support surface (61a) such that the first bone anchor (1) is configured to pivot in the single pivot plane defined by the pivot axis (C) and the longitudinal axis (I) of the support surface (61a) or
wherein the restraining structure (600b) is oriented at an angle to the longitudinal axis (I) of the support surface (61a), preferably an angle of 90°, such that the first bone anchor (1) is configured to pivot in the single pivot plane defined by the pivot axis (C) and an axis (k) transverse, preferably perpendicular, to the longitudinal axis (I) of the support surface (61a).

4. The coupling device of one of claims 1 to 3, wherein the pressure member (6') defines a support surface (61a) for the rod (100) and wherein the restraining structure comprises a first pair of recesses (600a) aligned with a longitudinal axis (I) of the support surface (61a) and a second pair of recesses (600b) oriented at an angle to the longitudinal axis (I) of the support surface (61a), preferably an angle of 90°, such that the coupling device is configured to connect to a third bone anchor (1") in such a manner that the third bone anchor (1") is prevented from pivoting with respect to the pivot axis (C).

5. The coupling device of one of claims 1 to 4, wherein the pressure member (6, 6') further comprises two upstanding legs (62) and wherein at least one of the legs (62) comprises a finger (64) that is configured to cooperate with a portion of the receiving part (5, 5') to prevent rotation of the pressure member, preferably wherein the finger (64) comprises an axially extending groove (64b) which a projection of the receiving part is configured to engage.

6. The coupling device of one of claims 1 to 5, wherein the receiving part (5, 5') comprises a first end (5a) and an opposite second end (5b), a recess (53) for receiving the rod (100), and an opening (52b) at the second end (5b) a greatest width of which is greater than a greatest outer diameter of the head (3, 3') of the first bone anchor (1) or the second bone anchor (1') to allow the head (3, 3') to be inserted from the second end (5b) through the opening (52b).

7. The coupling device of one of claims 1 to 6, further comprising an insert member (7, 7') that is positionable at least partially in the pressure member (6) and movable from a first position to a second position to exert an adjustable pressure on an inserted head (3, 3') to clamp the head (3, 3') relative to the receiving part (5)
wherein optionally the insert member (7') comprises a projection (74) that is configured to engage a recess (32) in a free end surface (3a) of the head (3, 3') to provide a form-fit connection between the projection (74) and the head (3, 3') that prevents the first bone anchor (1) or the second bone anchor (1') from pivoting relative to the receiving part (5).

8. A bone anchoring system comprising a coupling device of one of claims 1 to 7 and at least two of a first bone anchor (1) of a first type, a second bone anchor (1') of a second type, and a third bone anchor (1") of a third type,
wherein the first bone anchor (1) comprises a matching restraining structure (4) such that the first bone anchor (1) is configured to pivot with respect to the pivot axis (C) in a single plane,
wherein the second bone anchor (1') is configured to pivot with respect to the pivot axis in multiple planes, and
wherein the third bone anchor (1") comprises a matching restraining structure (4') such that the third bone anchor (1") is prevented from pivoting with respect to the pivot axis (C).

9. The bone anchoring system of claim 8, wherein the head (3) of the first bone anchor (1) has a substantially spherically-shaped outer surface portion and comprises a restraining structure (4) at the outer surface that is configured to cooperate with the restraining structure (58, 158a, 158b, 600, 600a, 600b) of the coupling device, preferably wherein the restraining structure (4) is a projection comprising at least one wing (4) that extends away from a shank axis (S) and that defines together with the shank axis (S) the single pivot plane

10. The bone anchoring system of claim 8 or 9, wherein the head (3) of the third bone anchor (1") has a substantially spherically-shaped outer surface portion and comprises a restraining structure (4') at the outer surface that is configured to cooperate with the restraining structure (158a, 158b, 600a, 600b) of the coupling device, preferably wherein the restraining structure (4') is a projection comprising at least two wings (4'), each wing extending away from a shank axis (S) and at least two of the wings being spaced apart from one another in a circumferential direction of the spherically-shaped outer surface portion of the head (3) at an angle different from 180°, preferably at an angle of 90°.

11. The bone anchoring system of one of claims 8 to 10, wherein the restraining structure (58, 158a, 600, 600a) of the coupling device (10, 10') is symmetrical to a plane that includes the pivot axis (C) and that is perpendicular to a longitudinal axis (L) of the rod receiving portion (53) and/or
wherein the restraining structure (158b, 600b) of the coupling device (10') is symmetrical to a plane that includes the pivot axis (C) and the longitudinal axis (L) of the rod receiving portion (53).

12. The bone anchoring system of one of claims 8 to 11, wherein the head (3') of the second bone anchor (1') has a uniform spherically-shaped outer surface portion that is free from a restraining structure.

13. The bone anchoring system of one of claims 8 to 12, wherein the head (3) of the first bone anchor (1) and the head (3') of the second bone anchor (1') comprises a free end (3a) with a recess (32) for engagement by an insert member (7) to prevent pivoting of the first bone anchor (1) or the second bone anchor (1').

14. An instrument for inserting a bone anchor into bone, the bone anchor being the first bone anchor (1) and/or the third bone anchor (1") of the bone anchoring system of one of claims 8 to 13, the bone anchor (1, 1") comprising a head (3) and a shank (2), wherein the head (3) has a substantially spherically-shaped outer surface portion and comprises a restraining structure (4, 4') at the outer surface that is configured to cooperate with the restraining structure (58, 158a, 158b, 600, 600a, 600b) of the coupling device (10, 10'), wherein the restraining structure (4, 4') of the bone anchor is a projection comprising at least one wing (4, 4') that extends away from a shank axis (S) of the shank (2), and the wing preferably extending parallel to the shank axis (S) along the spherically-shaped outer surface portion of the head (3),
wherein the instrument (200, 300) comprises an engagement structure comprising at least one groove (204, 304) configured to receive the at least one wing (4, 4') of the head (3) therein for coupling the instrument to the bone anchor (1, 1") so as to prevent rotation of the bone anchor relative to the instrument.

15. The instrument of claim 14, further comprising a spherically-shaped or U-shaped portion (203, 303) configured to engage the spherically-shaped outer surface portion of the head (3) of the bone anchor (1, 1") at least in a portion thereof and/or
wherein the restraining structure (4, 4') of the bone anchor comprises at least two wings spaced apart in a circumferential direction of the head (3) and the engagement structure of the instrument comprises at least two grooves (204, 304), each groove being configured to receive one of the wings.

16. The bone anchoring system of one of claims 8 to 13, further comprising the instrument (200, 300) of claim 14 or 15.

## Patentansprüche

1. Kopplungsvorrichtung zum Koppeln eines Stabs mit einem ersten Knochenanker (1) eines ersten Typs oder mit einem zweiten Knochenanker (1') eines zweiten Typs, wobei die Kopplungsvorrichtung (10, 10') umfasst:
ein Aufnahmeteil (5, 5') mit einem Stabaufnahmeabschnitt (53) zum Aufnehmen des Stabs (100) und einem Kopfaufnahmeabschnitt (52) zum schwenkbaren Aufnehmen eines Kopfs (3) des ersten Knochenankers (1) oder eines Kopfs (3') des zweiten Knochenankers (1'), so dass ein Schaft (2) des ersten oder des zweiten Knochenankers eine Mehrzahl von Winkelpositionen relativ zu einer Schwenkachse (C) einnehmen kann, die sich durch den Kopfaufnahmeabschnitt (52) erstreckt, und
ein Druckelement (6, 6'), das dazu ausgebildet ist, Druck auf einen eingesetzten Kopf (3, 3') des ersten Knochenankers (1) oder des zweiten Knochenankers (1') auszuüben, um den Kopf (3, 3') in einer bestimmten Winkelposition zu klemmen;
wobei die Kopplungsvorrichtung (10, 10') eine Rückhaltestruktur (58, 158a, 158b, 600, 600a, 600b) umfasst, mittels der die Kopplungsvorrichtung (10, 10') dazu ausgebildet ist, mit dem ersten Knochenanker (1) derart zusammenzuwirken, dass der erste Knochenanker (1) dazu ausgebildet ist, in Bezug auf die Schwenkachse (C) in einer einzigen Ebene zu schwenken, oder mit dem zweiten Knochenanker (1') derart zusammenzuwirken, dass der zweite Knochenanker (1') dazu ausgebildet ist, in Bezug auf die Schwenkachse (C) in mehreren Ebenen zu schwenken,
**dadurch gekennzeichnet, dass** die Rückhaltestruktur eine erste Ausnehmung (600, 600a) umfasst, die in dem Druckelement (6, 6') vorgesehen ist und die dazu ausgebildet ist, mit einer passenden Rückhaltestruktur (4) des ersten Knochenankers (1) zusammenzuwirken, um ein Schwenken des ersten Knochenankers (1) in der einzigen Ebene zu ermöglichen, und die Rückhaltestruktur eine zweite Ausnehmung (58, 158a, 158b) an dem Aufnahmeteil (5, 5') umfasst, die der ersten Ausnehmung an dem Druckelement entspricht und die dazu ausgebildet ist, mit dem ersten Knochenanker (1) zusammenzuwirken.

2. Kopplungsvorrichtung nach Anspruch 1, wobei das Druckelement (6, 6') einen Kopfaufnahmeabschnitt (66) umfasst, der einen eingesetzten Kopf (3, 3') des ersten Knochenankers (1) oder des zweiten Knochenankers (1') von der Seite her umschließt, wobei der Kopfaufnahmeabschnitt (66) eine untere Öffnung (67) zum Durchführen des Schafts (2) definiert, wobei die Rückhaltestruktur (600, 600a) an die untere Öffnung (67) angrenzt.

3. Kopplungsvorrichtung nach Anspruch 1 oder 2, wobei das Druckelement (6, 6') eine Auflagefläche (61a) für den Stab (100) definiert und wobei die Rückhaltestruktur (600, 600a) mit einer Längsachse (l) der Auflagefläche (61a) derart ausgerichtet ist, dass der erste Knochenanker (1) dazu ausgebildet ist, in der einzigen Schwenkebene zu schwenken, die durch die Schwenkachse (C) und die Längsachse (l) der Auflagefläche (61a) definiert ist, oder
wobei die Rückhaltestruktur (600b) in einem Winkel zu der Längsachse (l) der Auflagefläche (61a), vorzugsweise einem Winkel von 90°, derart ausgerichtet ist, dass der erste Knochenanker (1) dazu ausgebildet ist, in der einzigen Schwenkebene zu schwenken, die durch die Schwenkachse (C) und eine Achse (k) quer, vorzugsweise senkrecht, zu der Längsachse (l) der Auflagefläche (61a) definiert ist.

4. Kopplungsvorrichtung nach einem der Ansprüche 1 bis 3, wobei das Druckelement (6') eine Auflagefläche (61a) für den Stab (100) definiert und wobei die Rückhaltestruktur ein erstes Paar von Ausnehmungen (600a), die mit einer Längsachse (l) der Auflagefläche (61a) ausgerichtet sind, und ein zweites Paar von Ausnehmungen (600b) umfasst, die in einem Winkel zu der Längsachse (l) der Auflagefläche (61a), vorzugsweise einem Winkel von 90°, derart ausgerichtet sind, dass die Kopplungsvorrichtung dazu ausgebildet ist, mit einem dritten Knochenanker (1") derart verbunden zu sein, dass der dritte Knochenanker (1") daran gehindert wird, in Bezug auf die Schwenkachse (C) zu schwenken.

5. Kopplungsvorrichtung nach einem der Ansprüche 1 bis 4, wobei das Druckelement (6, 6') weiter zwei aufrecht stehende Schenkel (62) umfasst und wobei mindestens einer der Schenkel (62) einen Finger (64) umfasst, der dazu ausgebildet ist, mit einem Abschnitt des Aufnahmeteils (5, 5') zusammenzuwirken, um eine Drehung des Druckelements zu verhindern, vorzugsweise wobei der Finger (64) eine sich axial erstreckende Nut (64b) umfasst, mit der ein Vorsprung des Aufnahmeteils in Eingriff sein kann.

6. Kopplungsvorrichtung nach einem der Ansprüche 1 bis 5, wobei das Aufnahmeteil (5, 5') ein erstes Ende (5a) und ein gegenüberliegendes zweites Ende (5b), eine Ausnehmung (53) zum Aufnehmen des Stabs (100) und eine Öffnung (52b) an dem zweiten Ende (5b) umfasst, deren größte Breite größer als ein größter Außendurchmesser des Kopfs (3, 3') des ersten Knochenankers (1) oder des zweiten Knochenankers (1') ist, um zu ermöglichen, dass der Kopf (3, 3') von dem zweiten Ende (5b) durch die Öffnung (52b) eingesetzt wird.

7. Kopplungsvorrichtung nach einem der Ansprüche 1 bis 6, weiter umfassend ein Einsatzelement (7, 7'), das zumindest teilweise in dem Druckelement (6) positionierbar ist und von einer ersten Position in eine zweite Position bewegbar ist, um einen einstellbaren Druck auf einen eingesetzten Kopf (3, 3') auszuüben, um den Kopf (3, 3') relativ zu dem Aufnahmeteil (5) zu klemmen,
wobei optional das Einsatzelement (7') einen Vorsprung (74) umfasst, der dazu ausgebildet ist, mit einer Ausnehmung (32) in einer freien Endfläche (3a) des Kopfs (3, 3') in Eingriff zu sein, um eine formschlüssige Verbindung zwischen dem Vorsprung (74) und dem Kopf (3, 3') bereitzustellen, die verhindert, dass der erste Knochenanker (1) oder der zweite Knochenanker (1') relativ zu dem Aufnahmeteil (5) schwenkt.

8. Knochenverankerungssystem, umfassend eine Kopplungsvorrichtung nach einem der Ansprüche 1 bis 7 und mindestens zwei von einem ersten Knochenanker (1) eines ersten Typs, einem zweiten Knochenanker (1') eines zweiten Typs und einem dritten Knochenanker (1") eines dritten Typs,
wobei der erste Knochenanker (1) eine passende Rückhaltestruktur (4) umfasst, derart, dass der erste Knochenanker (1) dazu ausgebildet ist, in Bezug auf die Schwenkachse (C) in einer einzigen Ebene zu schwenken,
wobei der zweite Knochenanker (1') dazu ausgebildet ist, in Bezug auf die Schwenkachse in mehreren Ebenen zu schwenken, und
wobei der dritte Knochenanker (1") eine passende Rückhaltestruktur (4') umfasst, derart, dass der dritte Knochenanker (1") daran gehindert wird, in Bezug auf die Schwenkachse (C) zu schwenken.

9. Knochenverankerungssystem nach Anspruch 8, wobei der Kopf (3) des ersten Knochenankers (1) einen im Wesentlichen kugelförmigen Außenflächenabschnitt aufweist und eine Rückhaltestruktur (4) an der Außenfläche umfasst, die dazu ausgebildet ist, mit der Rückhaltestruktur (58, 158a, 158b, 600, 600a, 600b) der Kopplungsvorrichtung zusammenzuwirken, vorzugsweise wobei die Rückhaltestruktur (4) ein Vorsprung ist, der mindestens einen Flügel (4) umfasst, der sich von einer Schaftachse (S) weg erstreckt und der zusammen mit der Schaftachse (S) die einzige Schwenkebene definiert.

10. Knochenverankerungssystem nach Anspruch 8 oder 9, wobei der Kopf (3) des dritten Knochenankers (1") einen im Wesentlichen kugelförmigen Außenflächenabschnitt aufweist und eine Rückhaltestruktur (4') an der Außenfläche umfasst, die dazu ausgebildet ist, mit der Rückhaltestruktur (158a, 158b, 600a, 600b) der Kopplungsvorrichtung zusammenzuwirken, vorzugsweise wobei die Rückhaltestruktur (4') ein Vorsprung ist, der mindestens zwei Flügel (4') umfasst, wobei sich jeder Flügel von einer Schaftachse (S) weg erstreckt und mindestens zwei der Flügel in einer Umfangsrichtung des kugelförmigen Außenflächenabschnitts des Kopfs (3) in einem Winkel, der von 180° verschieden ist, vorzugsweise in einem Winkel von 90°, voneinander beabstandet sind.

11. Knochenverankerungssystem nach einem der Ansprüche 8 bis 10, wobei die Rückhaltestruktur (58, 158a, 600, 600a) der Kopplungsvorrichtung (10, 10') symmetrisch zu einer Ebene ist, die die Schwenkachse (C) enthält und die senkrecht zu einer Längsachse (L) des Stabaufnahmeabschnitts (53) ist, und/oder
wobei die Rückhaltestruktur (158b, 600b) der Kopplungsvorrichtung (10') symmetrisch zu einer Ebene ist, die die Schwenkachse (C) und die Längsachse (L) des Stabaufnahmeabschnitts (53) enthält.

12. Knochenverankerungssystem nach einem der Ansprüche 8 bis 11, wobei der Kopf (3') des zweiten Knochenankers (1') einen gleichförmigen kugelförmigen Außenflächenabschnitt aufweist, der frei von einer Rückhaltestruktur ist.

13. Knochenverankerungssystem nach einem der Ansprüche 8 bis 12, wobei der Kopf (3) des ersten Knochenankers (1) und der Kopf (3') des zweiten Knochenankers (1') ein freies Ende (3a) mit einer Ausnehmung (32) zum Eingriff durch ein Einsatzelement (7) umfassen, um ein Schwenken des ersten Knochenankers (1) oder des zweiten Knochenankers (1') zu verhindern.

14. Instrument zum Einsetzen eines Knochenankers in Knochen, wobei der Knochenanker der erste Knochenanker (1) und/oder der dritte Knochenanker (1") des Knochenverankerungssystems nach einem der Ansprüche 8 bis 13 ist, wobei der Knochenanker (1, 1") einen Kopf (3) und einen Schaft (2) umfasst, wobei der Kopf (3) einen im Wesentlichen kugelförmigen Außenflächenabschnitt aufweist und eine Rückhaltestruktur (4, 4') an der Außenfläche umfasst, die dazu ausgebildet ist, mit der Rückhaltestruktur (58, 158a, 158b, 600, 600a, 600b) der Kopplungsvorrichtung (10, 10') zusammenzuwirken, wobei die Rückhaltestruktur (4, 4') des Knochenankers ein Vorsprung ist, der mindestens einen Flügel (4, 4') umfasst, der sich von einer Schaftachse (S) des Schafts (2) weg erstreckt, und wobei sich der Flügel vorzugsweise parallel zu der Schaftachse (S) entlang des kugelförmigen Außenflächenabschnitts des Kopfs (3) erstreckt,
wobei das Instrument (200, 300) eine Eingriffsstruktur umfasst, die mindestens eine Nut (204, 304) umfasst, die dazu ausgebildet ist, den mindestens einen Flügel (4, 4') des Kopfs (3) darin aufzunehmen, um das Instrument mit dem Knochenanker (1, 1") zu koppeln, um eine Drehung des Knochenankers relativ zu dem Instrument zu verhindern.

15. Instrument nach Anspruch 14, weiter umfassend einen kugelförmigen oder U-förmigen Abschnitt (203, 303), der dazu ausgebildet ist, mit dem kugelförmigen Außenflächenabschnitt des Kopfs (3) des Knochenankers (1, 1") zumindest abschnittsweise in Eingriff zu sein, und/oder
wobei die Rückhaltestruktur (4, 4') des Knochenankers mindestens zwei Flügel umfasst, die in einer Umfangsrichtung des Kopfs (3) voneinander beabstandet sind, und die Eingriffsstruktur des Instruments mindestens zwei Nuten (204, 304) umfasst, wobei jede Nut dazu ausgebildet ist, einen der Flügel aufzunehmen.

16. Knochenverankerungssystem nach einem der Ansprüche 8 bis 13, weiter umfassend das Instrument (200, 300) nach Anspruch 14 oder 15.

## Revendications

1. Dispositif de couplage pour coupler une tige à un premier ancrage osseux (1) d'un premier type ou à un second ancrage osseux (1') d'un second type, le dispositif de couplage (10, 10') comprenant :
une partie de réception (5, 5') ayant une partie de réception de tige (53) pour recevoir la tige (100) et une partie de réception de tête (52) pour recevoir de manière pivotante une tête (3) du premier ancrage osseux (1) ou une tête (3') du second ancrage osseux (1') de sorte qu'un manche (2) du premier ou du second ancrage osseux peut adopter une pluralité de positions angulaires par rapport à un axe de pivotement (C) s'étendant à travers la partie de réception de tête (52), et
un élément de pression (6, 6') qui est configuré pour exercer une pression sur une tête insérée (3, 3') du premier ancrage osseux (1) ou du second ancrage osseux (1') pour serrer la tête (3, 3') à une position angulaire particulière ;
dans lequel le dispositif de couplage (10, 10') comprend une structure de retenue (58, 158a, 158b, 600, 600a, 600b) au moyen de laquelle le dispositif de couplage (10, 10') est configuré pour coopérer avec le premier ancrage osseux (1) de sorte que le premier ancrage osseux (1) est configuré pour pivoter par rapport à l'axe de pivotement (C) dans un plan unique, ou avec le second ancrage osseux (1') de sorte que le second ancrage osseux (1') est configuré pour pivoter par rapport à l'axe de pivotement (C) dans de multiples plans,
**caractérisé en ce que** la structure de retenue comprend un premier évidement (600, 600a) prévu dans l'élément de pression (6, 6') qui est configuré pour coopérer avec une structure de retenue correspondante (4) du premier ancrage osseux (1) pour permettre le pivotement du premier ancrage osseux (1) dans le plan unique, et la structure de retenue comprend un second évidement (58, 158a, 158b) sur la partie de réception (5, 5'), qui correspond au premier évidement sur l'élément de pression et qui est configuré pour coopérer avec le premier ancrage osseux (1).

2. Dispositif de couplage selon la revendication 1, dans lequel l'élément de pression (6, 6') comprend une partie de réception de tête (66) enfermant une tête insérée (3, 3') du premier ancrage osseux (1) ou du second ancrage osseux (1') de côté, la partie de réception de tête (66) définissant une ouverture inférieure (67) pour laisser passer le manche (2), dans lequel la structure de retenue (600, 600a) est adjacente à l'ouverture inférieure (67).

3. Dispositif de couplage selon la revendication 1 ou 2, dans lequel l'élément de pression (6, 6') définit une surface de support (61a) pour la tige (100) et dans lequel la structure de retenue (600, 600a) est alignée avec un axe longitudinal (l) de la surface de support (61a) de sorte que le premier ancrage osseux (1) est configuré pour pivoter dans le plan de pivotement unique défini par l'axe de pivotement (C) et l'axe longitudinal (l) de la surface de support (61a) ou
dans lequel la structure de retenue (600b) est orientée selon un angle par rapport à l'axe longitudinal (l) de la surface de support (61a), de préférence un angle de 90°, de sorte que le premier ancrage osseux (1) est configuré pour pivoter dans le plan de pivotement unique défini par l'axe de pivotement (C) et un axe (k) transversal, de préférence perpendiculaire, à l'axe longitudinal (l) de la surface de support (61a).

4. Dispositif de couplage selon l'une des revendications 1 à 3, dans lequel l'élément de pression (6') définit une surface de support (61a) pour la tige (100) et dans lequel la structure de retenue comprend une première paire d'évidements (600a) alignés avec un axe longitudinal (l) de la surface de support (61a) et une seconde paire d'évidements (600b) orientés selon un angle par rapport à l'axe longitudinal (l) de la surface de support (61a), de préférence un angle de 90°, de sorte que le dispositif de couplage est configuré pour se raccorder à un troisième ancrage osseux (1") de telle manière que le troisième ancrage osseux (1") est empêché de pivoter par rapport à l'axe de pivotement (C).

5. Dispositif de couplage selon l'une des revendications 1 à 4, dans lequel l'élément de pression (6, 6') comprend en outre deux branches verticales (62) et dans lequel au moins l'une des branches (62) comprend un doigt (64) qui est configuré pour coopérer avec une partie de la partie de réception (5, 5') pour empêcher la rotation de l'élément de pression, de préférence dans lequel le doigt (64) comprend une rainure s'étendant axialement (64b) avec laquelle une saillie de la partie de réception est configurée pour s'engager.

6. Dispositif de couplage selon l'une des revendications 1 à 5, dans lequel la partie de réception (5, 5') comprend une première extrémité (5a) et une seconde extrémité opposée (5b), un évidement (53) pour recevoir la tige (100), et une ouverture (52b) au niveau de la seconde extrémité (5b) dont la plus grande largeur est supérieure au plus grand diamètre extérieur de la tête (3, 3') du premier ancrage osseux (1) ou du second ancrage osseux (1') pour permettre à la tête (3, 3') d'être insérée à partir de la seconde extrémité (5b) à travers l'ouverture (52b).

7. Dispositif de couplage selon l'une des revendications 1 à 6, comprenant en outre un élément d'insertion (7, 7') qui peut être positionné au moins partiellement dans l'élément de pression (6) et mobile d'une première position à une seconde position pour exercer une pression réglable sur une tête insérée (3, 3') pour serrer la tête (3, 3') par rapport à la partie de réception (5)
dans lequel facultativement l'élément d'insertion (7') comprend une saillie (74) qui est configurée pour s'engager avec un évidement (32) dans une surface d'extrémité libre (3a) de la tête (3, 3') pour fournir une connexion par complémentarité de forme entre la saillie (74) et la tête (3, 3') qui empêche le premier ancrage osseux (1) ou le second ancrage osseux (1') de pivoter par rapport à la partie de réception (5).

8. Système d'ancrage osseux comprenant un dispositif de couplage selon l'une des revendications 1 à 7 et au moins deux parmi un premier ancrage osseux (1) d'un premier type, un second ancrage osseux (1') d'un second type, et un troisième ancrage osseux (1") d'un troisième type,
dans lequel le premier ancrage osseux (1) comprend une structure de retenue correspondante (4) de sorte que le premier ancrage osseux (1) est configuré pour pivoter par rapport à l'axe de pivotement (C) dans un plan unique,
dans lequel le second ancrage osseux (1') est configuré pour pivoter par rapport à l'axe de pivotement dans de multiples plans, et
dans lequel le troisième ancrage osseux (1") comprend une structure de retenue correspondante (4') de sorte que le troisième ancrage osseux (1") est empêché de pivoter par rapport à l'axe de pivotement (C).

9. Système d'ancrage osseux selon la revendication 8, dans lequel la tête (3) du premier ancrage osseux (1) a une partie de surface externe de forme sensiblement sphérique et comprend une structure de retenue (4) au niveau de la surface externe qui est configurée pour coopérer avec la structure de retenue (58, 158a, 158b, 600, 600a, 600b) du dispositif de couplage, de préférence dans lequel la structure de retenue (4) est une saillie comprenant au moins une aile (4) qui s'étend à distance d'un axe de manche (S) et qui définit conjointement avec l'axe de manche (S) le plan de pivotement unique.

10. Système d'ancrage osseux selon la revendication 8 ou 9, dans lequel la tête (3) du troisième ancrage osseux (1") a une partie de surface externe de forme sensiblement sphérique et comprend une structure de retenue (4') au niveau de la surface externe qui est configurée pour coopérer avec la structure de retenue (158a, 158b, 600a, 600b) du dispositif de couplage, de préférence dans lequel la structure de retenue (4') est une saillie comprenant au moins deux ailes (4'), chaque aile s'étendant à distance d'un axe de manche (S) et au moins deux des ailes étant espacées l'une de l'autre dans une direction circonférentielle de la partie de surface externe de forme sphérique de la tête (3) selon un angle différent de 180°, de préférence selon un angle de 90°.

11. Système d'ancrage osseux selon l'une des revendications 8 à 10, dans lequel la structure de retenue (58, 158a, 600, 600a) du dispositif de couplage (10, 10') est symétrique à un plan qui comprend l'axe de pivotement (C) et qui est perpendiculaire à un axe longitudinal (L) de la partie de réception de tige (53) et/ou
dans lequel la structure de retenue (158b, 600b) du dispositif de couplage (10') est symétrique à un plan qui comprend l'axe de pivotement (C) et l'axe longitudinal (L) de la partie de réception de tige (53).

12. Système d'ancrage osseux selon l'une des revendications 8 à 11, dans lequel la tête (3') du deuxième ancrage osseux (1') a une partie de surface externe de forme sphérique uniforme qui est dépourvue d'une structure de retenue.

13. Système d'ancrage osseux selon l'une des revendications 8 à 12, dans lequel la tête (3) du premier ancrage osseux (1) et la tête (3') du deuxième ancrage osseux (1') comprennent une extrémité libre (3a) avec un évidement (32) pour l'engagement par un élément d'insertion (7) pour empêcher le pivotement du premier ancrage osseux (1) ou du second ancrage osseux (1').

14. Instrument pour insérer un ancrage osseux dans l'os, l'ancrage osseux étant le premier ancrage osseux (1) et/ou le troisième ancrage osseux (1") du système d'ancrage osseux selon l'une des revendications 8 à 13, l'ancrage osseux (1, 1") comprenant une tête (3) et un manche (2), dans lequel la tête (3) a une partie de surface externe de forme sensiblement sphérique et comprend une structure de retenue (4, 4') au niveau de la surface externe qui est configurée pour coopérer avec la structure de retenue (58, 158a, 158b, 600, 600a, 600b) du dispositif de couplage (10, 10'), dans lequel la structure de retenue (4, 4') de l'ancrage osseux est une saillie comprenant au moins une aile (4, 4') qui s'étend à distance d'un axe de manche (S) du manche (2), et l'aile s'étendant de préférence parallèlement à l'axe de manche (S) le long de la partie de surface externe de forme sphérique de la tête (3),
dans lequel l'instrument (200, 300) comprend une structure d'engagement comprenant au moins une rainure (204, 304) configurée pour recevoir l'au moins une aile (4, 4') de la tête (3) dans celle-ci pour coupler l'instrument à l'ancrage osseux (1, 1") de manière à empêcher la rotation de l'ancrage osseux par rapport à l'instrument.

15. Instrument selon la revendication 14, comprenant en outre une partie de forme sphérique ou en forme de U (203, 303) configurée pour s'engager avec la partie de surface externe de forme sphérique de la tête (3) de l'ancrage osseux (1, 1") au moins dans une partie de celle-ci et/ou
dans lequel la structure de retenue (4, 4') de l'ancrage osseux comprend au moins deux ailes espacées dans une direction circonférentielle de la tête (3) et la structure d'engagement de l'instrument comprend au moins deux rainures (204, 304), chaque rainure étant configurée pour recevoir l'une des ailes.

16. Système d'ancrage osseux selon l'une des revendications 8 à 13, comprenant en outre l'instrument (200, 300) selon la revendication 14 ou 15.
